# EUROPEAN PATENT SPECIFICATION

(11) **EP 4 456 836 B1**
(45) Date of publication and mention of the grant of the patent: **04.11.2026**
(21) Application number: 22839496.1
(22) Date of filing: 21.12.2022
(51) Int. Cl.: A61F 2/68, A61F 2/64

(54) **CONTROL SYSTEM FOR PROSTHETIC DEVICE USING A MAGNETORHEOLOGICAL ACTUATOR**
STEUERUNGSSYSTEM FÜR PROTHETISCHE VORRICHTUNG MIT MAGNETORHEOLOGISCHEM AKTUATOR
SYSTÈME DE COMMANDE POUR DISPOSITIF PROTHÉTIQUE UTILISANT UN ACTIONNEUR MAGNÉTORHÉOLOGIQUE

(30) Priority: 28.12.2021 US 202163266084 P
(43) Date of publication of application: 06.11.2024
(73) Proprietor: Össur Iceland ehf, 110 Reykjavik (IS)
(72) Inventor: EINARSSON, Arni, 110 Reykjavík (IS); GANSEIJ, Maarten, 110 Reykjavík (IS); GUDMUNDSDOTTIR, Anna, 110 Reykjavík (IS); SVERRISSON, Atli Orn, 110 Reykjavík (IS)
(74) Representative: Invent Horizon IP
(86) International application number: PCT/IB2022/062647
(87) International publication number: WO 2023/126787

(56) References cited:
- WO-A1-2021/055851
- US-A1- 2002 052 663
- US-A1- 2014 200 680
- US-A1- 2016 074 180
- US-A1- 2020 022 823

## Description

### Field

The present disclosure relates to a control system for prosthetic devices, and more particularly to a control system for prosthetic devices that employ a magnetorheological ("MR") actuator.

### Description of the Related Art

Modern lower-limb prosthetic devices are leveraging the capabilities offered by microprocessors and high-power density actuators, such as hydraulic, pneumatic, or mechanical actuators, to bridge the gaps that have been associated with the traditional technologies used in prosthetic devices. The addition of sensors to the prosthetic devices, combined with on-board processing power, extends the range of performance observed on traditional devices by allowing them to dynamically adapt to changing environment and use conditions, as well as to dynamically modify the actuator's characteristics to implement an optimal system response.

Increases in performance of the lower-limb prosthetic device also increase the demands on the control system. While the traditional approaches relied on simplistic control mechanisms relying heavily on user inputs, modern approaches are targeted at reducing the burden on the end-user, while providing a wider range of functionalities and more natural coupling to the user intent.

WO 2021/055851 A1 discloses in one example a prosthetic or orthotic device that can include a controller and an actuator. The actuator can be implemented as a rotary MR knee actuator that includes MR fluid. The MR fluid is a field responsive (FR) fluid or medium that undergoes a rheology or viscosity change which is dependent on the magnitude of an applied magnetic field.

### SUMMARY

The following disclosure describes non-limiting embodiments of specific aspects of a control system for interpreting and managing the end-user's intent in a microprocessor-controlled knee prosthetic device where a shear type MR actuator is used.

Due to the complex and variable nature of human locomotion, the control system is advantageously structured in such a way as to make optimal use of the data collected by the embedded sensors in a prosthetic device. Additionally, use of a layered control system where various data abstraction levels are addressed in independent, but coupled, layers advantageously allows formalizing the data flow and determining the scope of each specific control loop. More specifically, the layered control system addresses three main tasks: Intent Recognition, Intent Management, and Actuator Control.

This disclosure describes the overall control system through its constituent parts and directs specific attention towards the inventive aspects of the control system. For example, a Luenberger observer is used for estimating knee joint velocity instead of a specific velocity sensor or lesser performing process such as time differentiation or filtering. Additionally, a control system with a heuristic torque set-point component using an angle dependent component is added on top of the basic velocity regulation control loop to advantageously control the function of a MR actuated knee prosthesis.

### BRIEF DESCRIPTION OF THE DRAWINGS

Figure 1 shows a perspective view of a microprocessor-controlled knee prosthetic using a shear-type MR actuator.
Figure 2 presents a block diagram of a control system for a microprocessor-controlled knee prosthetic using a MR actuator.
Figure 3 presents a block diagram associated with a layered control system architecture for prosthetic devices.
Figure 4 presents a block diagram showing the functional relationship and data flow of the state-machine used to implement the layered control system presented in Figure 3.
Figure 5 presents the general structure of a non-specific phase state machine.
Figure 6 presents the general structure of a non-specific subphase state machine.
Figure 7 presents the general structure of a non-specific activity state machine.
Figure 8 presents a block diagram of an example control scheme used to manage a prosthetic knee using MR actuator behaviour.
Figure 8A presents a block diagram of another example control scheme used to manage a prosthetic knee using MR actuator behaviour.
Figure 9 presents a block diagram associated with a non-specific implementation of a Luenberger observer used for computing actuator speed estimation in a microprocessor-controlled MR knee prosthetic device.
Figure 10 presents various embodiments of static torque characteristics for use as an input in a microprocessor-controlled knee prosthetic using a MR actuator.
Figure 11 presents various embodiments of Angle Dependent Component Torque characteristics for use as an input in a microprocessor-controlled knee prosthetic using a MR actuator.
Figure 12 presents a comparison between using a Luenberger Observer to calculate velocity versus traditional methods.

### DETAILED DESCRIPTION

The fundamental requirement for a control system to be used with a lower-limb prosthetic device is the ability to address three different types of tasks, which are different in nature, but all coupled in the way that they interact together to create an optimal combination of user, hardware, and firmware interaction. Firstly, it is required to identify what locomotion task the user is trying to complete. Human locomotion is composed of a variety of different tasks, which all have specific aspects that need to be addressed differently by the prosthetic device. Intent identification or locomotion task identification relies on embedded sensors that monitor the environment, the user's residual limb kinematics or the kinematics and/or kinetics resulting from the interaction between the user and the device. Combining the gathered data advantageously allows estimating the user's intent with good accuracy.

The second aspect of a modern control system is intent management. Once the most likely user intent or locomotion task at hand is identified, the system must then decide on which behavior the prosthetic device should adopt to properly support that task or intent. While traditional lower-limb prosthetic devices could only generate a limited number of behaviors, modern actuators can be leveraged to implement a much wider variety of behaviours that align more naturally with what would be observed in the human lower-limb joint. While this greatly increases the possibilities and refinement of the solution provided to the end-user, it also requires a more refined definition for the behaviors. Furthermore, great care must be directed at ensuring that the advanced behaviors integrate seamlessly with the end-user's motions and expectations.

Thirdly, the intent management strategy must be implemented with high accuracy with the actuator comprised in the system. Implementation of the strategy in the form of an action plan or actuator response then relies on a motion control scheme that controls the actuator. The specific combination of the motion control scheme and the actuator dynamic response must replicate the targeted human articulation behavior to ensure satisfactory performance and seamless integration with the user's locomotion. Motion control schemes are really bridging the gap between the detailed biomechanical requirements defining the general control strategy and the actuator characteristics, with the objective of having the combination of the two generate something where hard characteristics of the mechanical componentry are transformed into the smooth and compliant nature of the human anatomy.

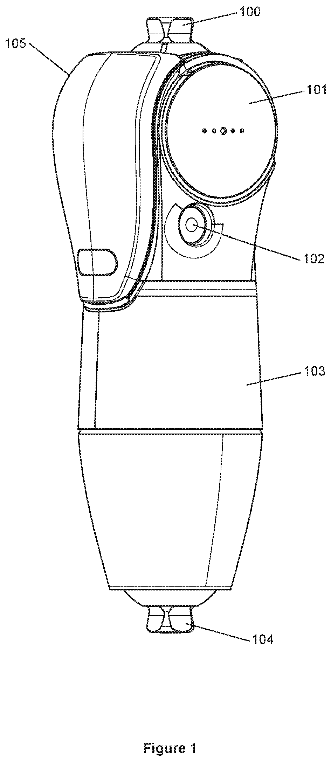

Figure 1 shows an embodiment of a microprocessor-controlled knee prosthetic device where a shear-type rotary MR actuator is used. This embodiment is a modular component targeted at being assembled with other modular components to create a complete prosthetic leg. Proximal Connector 100 is used to structurally connect the knee prosthetic device with the residual limb attachment system (not shown). The knee prosthetic device embodiment is non-specific to the type of residual limb attachment system. Socket systems relying on vacuum or mechanical fastening (e.g., pin liner suspension system), or osteointegration adapters are examples of possible interface solutions. The prosthetic knee integrates the rotary shear-type actuator **101** in the knee rotation axis area, allowing the thigh mounted segment and proximal connector **100** to rotate with respect to the shank segment **103.** Shank segment **103** is used to house the electronic components and battery required to implement the control electronics, actuator driver, on-board sensors and other various support systems that require protection from the environment and possible impact with objects. Mechanical lock actuator **102** allows for locking the motion between the thigh and shank segments of the prosthetic knee. The shank segment is structurally connected to the distal connector **104,** which allows for the connection of the distal modules of the prosthetic leg assembly, namely the shank pylon, as well as the foot and ankle modules. Knee pad **105** is mounted at the front of the MR actuator **101** and the upper part of the shank segment **103** in view of providing protection to the components when the device is used for kneeling or other similar uses.

The control systems embodiment herein described is well suited to be deployed in this type of lower-limb prosthetic as it makes optimal use of the layered control components to implement the level of refined controls required by the MR actuator's characteristics. The control system could also be used with other types of lower-limb prosthetic or actuator technologies without really imparting to the scope of the current disclosure.

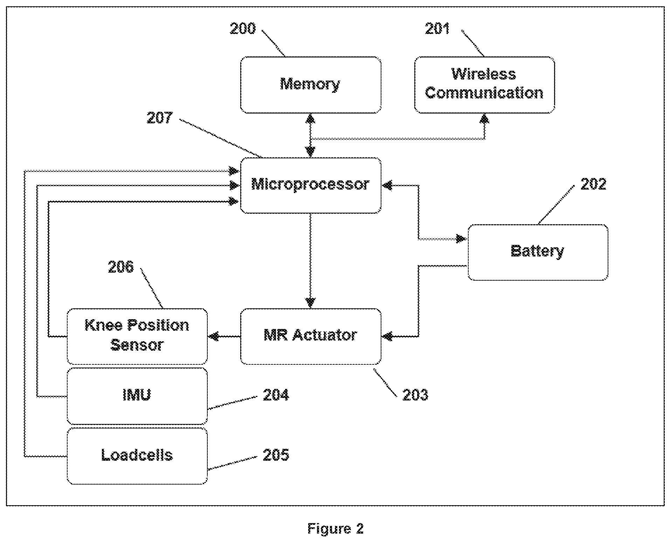

Figure 2 presents the general building blocks for a microprocessor-controlled prosthetic knee using a MR actuator, such as the example provided in Figure 1. Microprocessor **207** represents the core of the system's architecture, as it connects most of the other building blocks together and operates in such a way as to create a cohesive ensemble from the individual components and module. Microprocessor **207** is in operational communication with a memory block **200,** where information critical for the correct system operation can be stored and retrieved. Additionally, the microprocessor also manages the wireless communication module **201** to exchange information with external devices, such as mobile phones or tablets, where various type of applications can be used to retrieve information from the prosthetic device, trigger specific functions, or provide new data to be stored in the Memory **200.**

A battery **202** is present in the system to power the electronic components, such as the various sensors **204, 205,** and **206,** the microprocessor **207** and the actuator **203.** Various types of battery technologies can be used to power the control system. However, one has to consider the mobile nature of the system and the limited amount of weight that can be added to prosthetic systems before they lose their practicality. In that context, battery technologies providing high power and energy densities are considered better suited for this type of application. Additionally, secondary type battery technologies would reduce the operating costs of these devices by allowing the battery to be recharged, as well as minimize the environmental impact related to the device's operation.

Embedded sensors are integrated in the basic components of the control system, as they represent the main input source for implementing the various control loops and intent management functions. Loadcells **205** are one example of a type of sensor that can be used to monitor the interaction between the user wearing the prosthetic device and the immediate environment. Inertial Measurement Units (IMUs) **204** are one example of a sensor technology that can be integrated in the system to measure the kinematics of the end-user's residual limb or the prosthetic device. Knee position sensor **206** is one example of a type of sensor that can be used to measure knee joint kinematics directly, which are the result of the interaction between the end-user, the environment, and the prosthetic device actuator **203.** These sensors are functionally connected with the microprocessor **207,** which takes care of retrieving the information they provide and further processing it based on the definition of the detection and control algorithms.

Finally, the MR actuator **203** is functionally connected to and controlled by the microprocessor **207** based on the outcome of the data processing performed in firmware. As mentioned above, the MR actuator **203,** or any other type of actuator for that matter, implements the application-level strategies through motion control of the actuator itself. Implementation of the motion control strategies, combined with environmental and end-user interactions then causes a general system response that can be measured by the sensors, hence closing the feedback control loop.

Microprocessor **207** can be leveraged to implement a layered control system. Use of a layered control system architecture for the implementation of the application-level control mechanisms advantageously allows for simple and efficient organization of the data flow in the control system, as well as the creation of a data abstraction model that efficiently divides the tasks between the modules that are cohabiting in the device firmware.

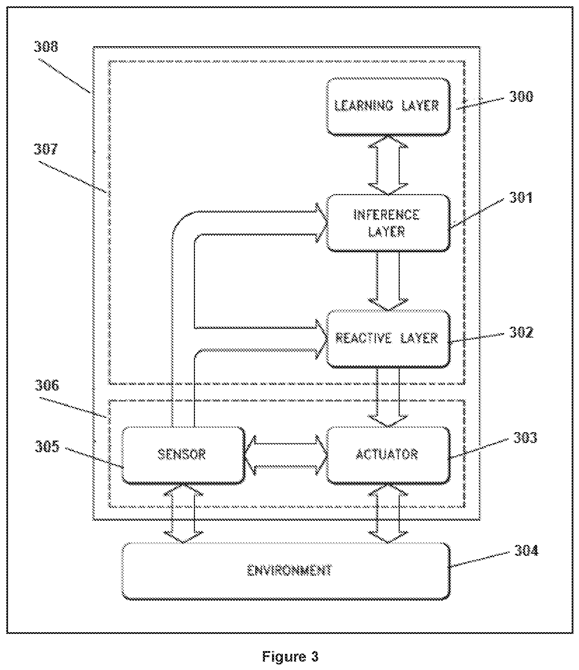

Figure 3 presents a non-limitative embodiment of a layered control system for use with lower-limb prosthetic devices. This type of layering and distribution of functions advantageously simplifies data management throughout the system, while also making use of the layered structure in a manner somewhat similar to what is observed in the human neuro-muscular control system, where both arc-reflex loops and motor commands generated from cognitive sources can be superimposed depending on the nature of the task at hand and the context in which they are taking place.

Prosthetic device **308** is composed of a control system **307** interfacing prosthetic device hardware components **306.** From their hardware nature, hardware components **306** directly interact with the environment **304** in which the prosthetic device is evolving, being either the end-user wearing the prosthetic device, the natural environment in which the end-user is operating the device, or a combination of both.

It is to be noted that for sake of clarity, hardware components of the prosthetic device have been limited to the components having a direct interaction with the control system. Hence, sensor components **305** and actuator components **303** are herein represented. Sensor components **305** are used to collect information about the prosthetic device and its environment and convert it to a data format that can be interpreted or managed by the microprocessor on which the layered control system **307** is implemented. Various types of sensing components can be used to sense the environment (i.e., force sensors, inertial sensors, kinematics sensors, etc.) or the prosthetic device internals itself (i.e., current sensor, force sensor, temperature sensor, etc.).

Data streams issued from the sensor components **305** are provided to the bottom two layers of the control system, namely the reactive layer **302** and the inference layer **301.** These two layers are directly involved in the intent detection and control of the prosthetic device behavior management. These layers make direct use of the information collected by the sensor components **305** to do so. Similar to the layering present in the human neuro-muscular control system, tasks are divided between the reactive layer **302** and the inference layer **301** based on the nature of the control task at hand, the level of information processing or abstraction required, and the time frame in which the control system action is required for the task at hand to be successfully completed. Generally, tasks associated with basic locomotion that require faster reaction from the control system would be addressed by the reactive layer, while tasks allowing for longer processing or that are not subject to strict time frames for completion would be performed by the inference layer. In comparison with the human neuro-muscular control system, the reactive layer takes care of the role executed by the arc-reflex loops, which address needs for fast reactive answers to pre-established stimuli. For example, direct management of the prosthetic device behavior when changes in loading level are observed would typically be a reactive layer task. Along the same lines, the inference layer addresses tasks performed by the neuro-motor cortex in the human neuro-muscular control system, where tasks requiring longer processing are handled. For example, processing of multiple data streams to determine a change in locomotion activity and extracting relevant information to implement the proper behaviors associated with the new locomotion activity is an inference layer level task.

Learning layer **300** is in functional communication with the inference layer **301** and is generally used to recursively improve the prosthetic device or control system performance as time passes and experience is built. In an analogous manner to the human cognitive function, the learning layer **301** adjusts the mapping of the decisional or control parameters to improve on long-term performance or adjust to specific characteristics of device usage by the end-user.

Finally, actuator components **303** execute the predefined behaviors or decisions generated by the inference layer **301** and reactive layer **302,** to realize the high-level goal of properly supporting the locomotion task at hand. It is to be noted that the relationship between the inference layer **301** and reactive layer **302** is both hierarchical and parallel in nature. While the inference layer **301** processes data of higher abstraction level and makes decisions that are then implemented through the reactive layer **302** engine, the reactive and inference layers are responsible for specific tasks which are not shared and where the final decision comes from the combination of the various contributions. In that sense, the vertical representation of the layered control system layers is more aligned with the level of data abstraction and complexity of decision making involved at each layer than it is representative of a hierarchical relationship between the layers.

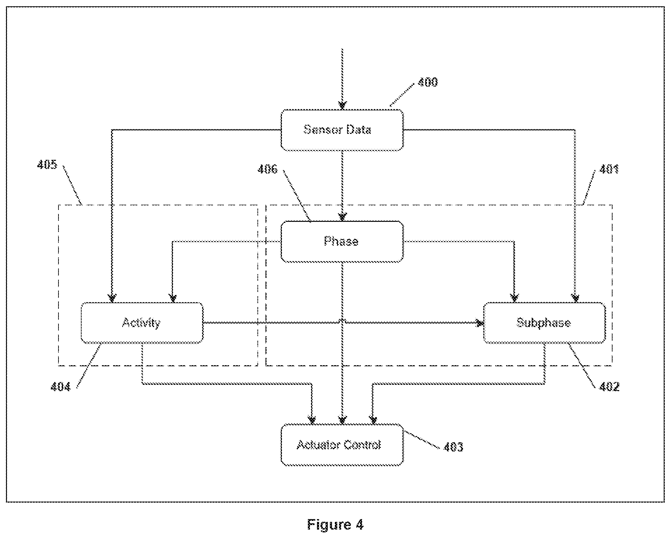

Figure 4 presents the functional relationship and data flow between the various state machines used to implement the layered control system previously introduced in Figure 3. Assuming an implementation of the layered control system where the learning layer **300** is not used, block diagrams of Figure 3 and Figure 4 are functionally equivalent, even if Figure 4 provides the additional level of details concerned with the actual content of the inference layer **301** and reactive layers **302** from Figure 3. Information collected from the embedded system is processed through the Sensor Data block **400,** which then feeds the relevant information, in correct representation type, to the various state machines part of the inference layer **405** and reactive layer **401.**

Reactive layer **401** contains the phase **406** and subphase **402** state machines. Both state machines rely on the information transferred from the sensor data block **400** to make decisions on the current state of the system from an application standpoint. In fact, combined with the inference layer **405** state machine, the role of these functional blocks is to identify the current gait activities being undertaken by the user, in view of noticing any changes and adapting the system behavior accordingly to the user's intent. These components form the intent identification and management aspect of the prosthetic device control system.

Basic identification of the gait activity taking place first starts with phase identification, as the phase is the most basic information on which the system relies. Phase identification is performed by the phase state machine **406** based on the provided sensor data stream. Decisions regarding the phase identification are then provided to the subphase state machine **402,** which then uses the information to further divide the respective phases into subphases, which are a more detailed breakdown of the phase from a gait perspective. Subphase state machines can make advantageous use of the activity information issued by the Activity state machine **404,** as subphase exact definition may be varied for each activity to properly represent the gait task. Additionally, phase information is provided to the activity state machine **404** and the actuator control block **403.**

The Activity state machine **404** uses the information coming from the Sensor Data **400,** as well as the Phase state machine **406** information to infer the activity currently being executed by the end-user, as well as to manage the transition between the activities.

Actuator Control **403** leverages the information issued from the Phase **406,** Subphase **402** and Activity **404** state machines to establish the action and behaviors required from the actuator included in the lower-limb prosthetic system. While the various state machines are based on a varying level of data abstraction and all have a specific scope, their operation is more or less parallel and the data flow between the state machines makes optimal use of that layered control system implementation.

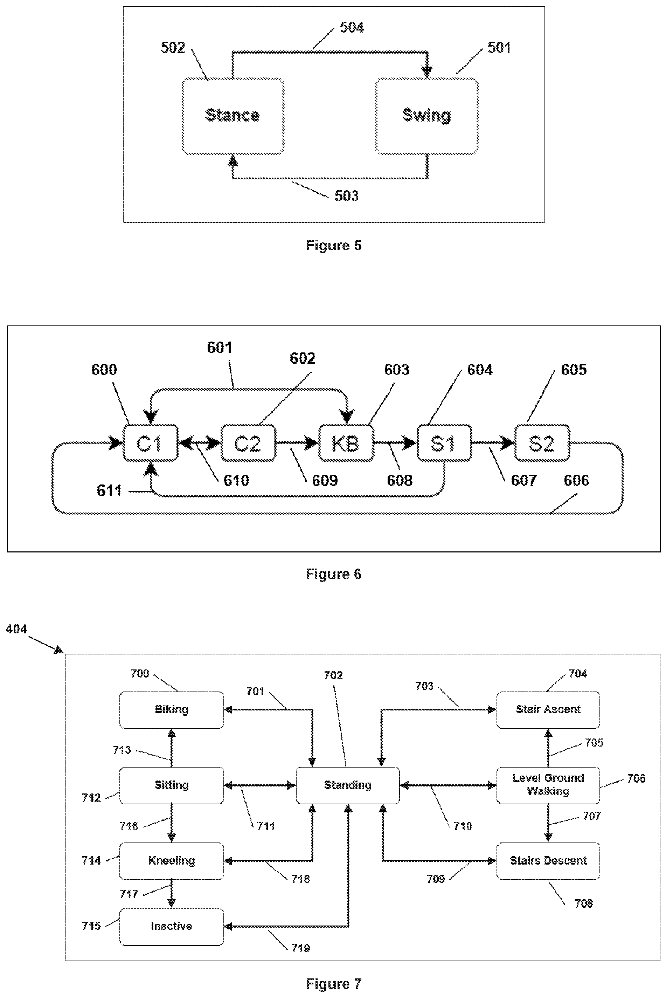

Figure 5 provides additional details on the Phase state machine previously introduced. From a human locomotion perspective, two phases are to be considered and are fundamental to the control of the lower-limb prosthetic device. The state machine is built around having a specific state for each phase. Swing Phase state **501** is characterized by the fact that the prosthetic limb is not carrying the user's weight or no contact between the prosthetic limb and the ground is observed during this state. For practical considerations, this state will be entered when a predefined set of conditions are met, typically coming from the Stance Phase state **502.** Stance Phase state **502** is effectively the counterpart of the Swing Phase state **501** and is characterized by the prosthetic lower limb being in contact with the ground or the prosthetic limb carrying the user's body weight. Similar to the Swing Phase state, Stance Phase state **502** will be entered when a predefined set of conditions are met, typically coming from the Swing Phase state **501.**

On system initialization, the phase state can be defined as either Stance Phase state **502** or Swing Phase state **501.** Once new data is issued by the sensors, state will be re-evaluated and the state machine will directly transition to the correct state by comparing the sensor data to the pre-established transition conditions for stance to swing transition **504** or swing to stance transition **503.** Due to the low latency required for the system to load new sensor data and the phase state to be evaluated, there is not a hard functional limitation in having the phase considered stance or swing by default. In one non-limitative embodiment of the phase state machine, the swing to stance transition **503** is based on the comparison of the axial load applied on the lower-limb prosthetic device and measured by the on-board sensors with pre-defined threshold. For example, if the loadcells **205** measure an axial load superior to 8kg, the state machine will make the current state be Stance Phase. Conversely, if the load applied on the lower-limb prosthetic device is measured to be less than 8kg, the phase will transition to Swing Phase. A variety of sensor embodiments can be used to support the phase state machine operation, such as, for example, loadcells, accelerometers, pressure sensors, displacement sensors, radar, inductive sensors, and resistive sensors. Additionally, stance to swing transition **504** and swing to stance transition **503** can be based on decision mechanisms other than the single thresholding comparison provided in the example above. Decision making processes such as multiple thresholding or majority voting could also be applied to define the transition criteria. Additionally, the various methods for deciding if the conditions are met for allowing the system to transition from one state to the other in the phase state machine could also consider multiple data streams at the same time or use sensor integration or fusion to build data streams containing data at a higher level of abstraction.

While the stance and swing phases are characteristic of human locomotion, they do not provide a very detailed segmentation of the behavior required by a lower-limb prosthetic, as this phase segmentation only describes the general configuration of the lower-limb and not the behavior of the various joints in a single phase. To achieve a level of gait control refinement as required by the end-user to achieve consistent, stable, and safe locomotion, additional granularity is required, such that the actuator behavior can also be adjusted in a more granular manner.

Figure 6 presents a subphase state machine embodiment that is particularly well suited for controlling knee prosthetic devices. While the number and the nature of the subphases can vary and does not present a limitative embodiment, the general approach is typically to consider the subphases needed to properly address the requirements of level walking activity and then to use these subphases, or a subset of them, to address the other locomotion activities, which can typically be addressed satisfactorily with a reduced number of subphases. While going for a lower number of subphases may make sense for devices using an actuator subsystem not allowing for refined controllability and going for too many subphases may increase the complexity of the control system without bringing any benefits to the overall performance, a correct number of subphases allows to make optimal use of the actuator controllability and address the specific biomechanical requirements of the gait activity in a satisfactory manner. In the specific case of the subphase state machine presented in Figure 6, use of 5 subphases is aligned with the general understanding of the knee's role in level walking as described in modern biomechanical analysis of the lower limb.

C1 subphase **600** represents the Contact 1 subphase of the gait cycle and consists of the level walking stance phase state starting from the occurrence of the contact of the prosthetic limb foot with the ground and where the knee is typically observed to flex under the weight of the end-user. This flexion is typically observed to provide a certain level of shock absorption following the occurrence of the contact between the prosthetic foot and the ground surface. To properly support the end-user's weight transfer to the prosthetic limb, the prosthetic knee actuator is required to provide braking torque or torque resisting the motion imposed to the knee joint by the end-user weight and momentum.

C2 subphase **602** represents the Contact 2 subphase of the gait cycle and consists of the level walking stance phase state where the knee is observed to extend, following the completion of the C1 subphase flexion motion. Extension motion occurs from a combination of the end-user forward momentum and the end-user residual limb hip extension. Proper support of the knee extension motion by the prosthetic device actuator requires an appropriate amount of resistive torque. Failure to provide enough resistive torque will cause the knee to quickly extend and hit the extension motion stop, which causes discomfort for the end-user. On the other hand, providing too much resistive torque will slow down the extension motion and require the end-user to use excessive force to extend the knee.

KB subphase **603** represents the knee break or pre-swing subphase of the level walking gait cycle and consists of the Stance Phase state where the knee joint is prepared to transition to Swing Phase. In that part of the level walking stance cycle, the user starts unloading the prosthetic limb and the residual limb thigh segment starts moving into hip flexion, after having reached maximum extension. Proper care must be directed in this subphase actuator control to allow for a smooth transition to swing, without hindering the end-user hip flexion, requiring the end-user to lift themself up on their sound foot (i.e., hip-hiking) or causing the prosthetic foot to stick to the floor and breaking the forward momentum of the end-user (late stance locking). To ensure proper synchronization with the user's motion and allow the user to control the whole KB subphase, the knee actuator is typically placed in a low resistance to motion state, which allows the end-user to properly control the knee joint behavior in this subphase.

S1 subphase **604** represents the Swing 1 subphase of the level walking gait cycle and consists in the swing phase state where the knee joint is actively flexing while not in contact with the ground, allowing the user to clear the ground while the lower limb is brought back in the proper configuration for the following step. Knee flexion under the momentum imparted to the shank segment through the thigh segment acceleration requires the knee joint actuator to present low resistive torque. Failure to properly leverage the residual limb momentum is typically observed to cause a slow flexion movement, which in turn is likely to cause toe-stubbing or fail at generating sufficient ground clearance while the hip is flexing the thigh segment. In both cases, the end-user's forward progression dynamics will be interrupted, creating a risk of fall or stumble, or reducing the overall efficiency of the walking gait pattern.

S2 subphase **605** represents the Swing 2 subphase of the level walking gait cycle and consists in the Swing Phase state where the knee joint is actively extending while not in contact with the ground, allowing the end-user to fully extend the knee joint in preparation for the upcoming transition to Stance Phase. Again in this subphase, it is required that the knee joint achieves sufficient velocity to ensure that the limb is fully extended ahead of the moment where the prosthetic foot would contact the ground surface, while also managing the joint velocity profile in such a way as to avoid the knee from hitting the extension stop, which is uncomfortable for the end-user and could negatively affect their capacity to transfer weight to the prosthetic limb in a timely manner. Properly supporting the S2 subphase requires the actuator to present a low resistance state for most of the subphase, followed by an increase in the actuator's resistance to motion to smoothly decelerate the joint in the last part of the extension motion. This type of actuator behavior can be obtained by using the subphase information combined with the knee position sensor data in the actuator control scheme.

C1 to C2 state transition **610** occurs when the knee joint behavior is observed to transition from stance flexion to stance extension, under the influence of the end-user. This transition is typically triggered through monitoring of the knee joint velocity direction and typically occurs from C1 to C2 during level walking. It is possible, however, that transition in the opposite direction (i.e., C2 to C1) could be observed in atypical circumstances or in other activities where knee joint extension is followed by knee joint flexion while in stance phase. Interruption in the level walking stance phase gait cycle could cause the extension motion to stop and the knee to start flexing again. Similarly, sudden stopping due to the presence of an obstacle or losing balance over the prosthetic foot could also generate this type of transition. On the other hand, other activities can be managed through these subphases and provide a more visual example of the bidirectionality of this specific transition. For example, stand to sit transfer is typically supported directly through the C1 subphase, since the knee is flexing in stance phase. Conversely, sit to stand transfer is typically directly supported through the C2 subphase. Since these transfer activities can be performed in any sequence, with or without really reaching the standing and sitting end points, this illustrates the need to support this transition in a bi-directional manner.

C2 to KB state transition **609** occurs when the user is observed to be ready to initiate the transition to swing phase and is again triggered through monitoring of the embedded sensors. This corresponds to the mid-stance to late-stance part of the level walking gait cycle, where the user's center of mass is observed to have moved anteriorly to the prosthetic foot and the hip is about to start flexing. Identification of this particular configuration of the user segments and body dynamics can be achieved by making sure that the knee is in an extended position and shows low velocity, which is indicative that the C2 subphase has really completed. Additionally, monitoring that the knee joint shows sufficient extension at that moment estimates the end-user's center of mass to be located anteriorly to the prosthetic foot. Finally, monitoring that sufficient shank sagittal plane rotational velocity is observed ensures that the user is actually showing forward progression momentum while rolling over the toe of the prosthetic foot and/or has started to flex the residual limb hip. As this transition is a rather dynamic one and actually requires the knee joint to be fully extended, it is typically considered as a one-way state transition. Exiting the KB state to return to an earlier stance phase state in level walking requires the knee to flex, which then matches the C1 subphase, and not the C2 subphase.

KB state **603** normal exit transition pattern during level walking gait is observed to be the KB to S1 state transition **608.** This transition is observed to take place when the user has completed the weight transfer to the contralateral limb, leaving the prosthetic limb now in swing phase. This transition is managed through the combination of two conditions monitored from the sensor data stream. For one, the loadcell signal has to indicate that the load on the prosthetic limb has dropped below the threshold value used to determine the phase. In other words, the phase has to be detected as being swing. Additionally, to prevent the occurrence of a false positive detection of a KB to S1 transition, a minimum flexion angle is required for the state transition to be executed. In a non-limitative embodiment, an angle of 15° is used. Use of the additional minimum flexion angle condition over simply using the threshold on the loadcell measurement provides the added benefits of helping to debounce the loadcell signal and making sure the user has committed to the transition before actually implementing the state change (i.e., residual limb hip flexion has started to take place, causing the knee to flex). Debouncing is required for cases where the load transferred to the ground through the prosthetic limb would be moving across the threshold value with small amplitude, causing the system to quickly cycle through the Stance and Swing Phases. Use of the minimum angle threshold minimizes that concern as selection of a correct threshold value ensures that the user has started flexing the residual limb, which greatly removes the capacity to be loading the prosthetic limb.

KB to C1 and C1 to KB bidirectional state transition **601** integrate two specific cases that are not typically observed during level walking gait but are often encountered in the general user population based on the high variability of the environment in which human locomotion takes place, causing deviation in the normal occurrence of gait events. Firstly, C1 to KB state transition is in fact equivalent to the C2 to KB state transition introduced above and would typically be observed in cases where the C1 to C2 transition would not be observed, mainly caused by the knee extension not taking place as per expected, but where KB transition is still observed to take place. For example, in the case where the user would enter C1 with a fully extended knee but not generate enough flexion torque to cause the knee to yield, or flex, under load would not meet the conditions to enter C2 from C1 but could still meet the conditions for the KB transition in late stance. This type of situation is often observed when users were trained to use simpler mechanical prosthetic knee technology, where they have to force the knee in hyperextension during stance phase to compensate for the device's lack of capacity to provide support.

Secondly, KB to C1 state transition is required to provide a recovery mechanism to the end-user and pre-empt some falls from being generated by the user failing at properly progressing through the normal gait events and subphases. Two main situations are observed in level walking where the KB to C1 transition is leveraged, and both are associated with a general situation where the observation of the sensor stream indicates that it would be safer for the end-user to have the knee provide support instead of the no-support joint behavior associated with the KB state. In one case, this transition would be triggered by the observation of the knee extension moment being too small and the knee joint flexion angle being too large. This situation represents a case where the end-user center of mass progression has stopped while KB was previously entered, leaving the center of mass posterior to the prosthetic foot and the end-user in an unstable position. This could be observed if the user is required to abruptly stop, or is pushed back, for example. In the other case, if the user loading of the prosthetic device is observed to increase while KB was already entered, this is again indicative that the end-user's center of mass is not progressing forward as expected and he is not transferring his weight away from the prosthetic limb like normally observed in KB.

S1 to S2 state transition **607** occurs when the knee joint has completed the level walking swing phase flexion motion and is observed to start extending. This inversion of the knee rotation direction is normally synchronized with the residual limb hip flexion motion completion and will position the lower limb in a configuration ready to support the coming prosthetic foot contact with the ground. This state transition is managed through the monitoring of the knee joint velocity direction, which is implemented through using a null velocity threshold to decide whether the current direction of motion has reached the value allowing for the transition to take place.

S1 to C1 state transition **611** is observed to occur in specific circumstances where sufficient load would be observed on the prosthetic device, through monitoring of the loadcell sensor read out, while the loadcell sensor read out is expected to be operating in Swing Phase. Occurrence of Stance Phase detection during swing flexion can be related to a variety of factors but all indicate a disruption of the normal level walking gait cycle which requires providing support to the end-user and preventing the knee from collapsing under the user. For example, presence of an obstacle interrupting the swing flexion motion and/or interrupting the end-user's forward progression momentum could easily result in this transition taking place. Obstacles such as curbs, shrubs, tall grass, or snow are typical examples of obstacles encountered in daily living. Similar to the KB to C1 case previously introduced, situations where the user would be pushed back (e.g., from pushing against a heavy door, or bumping into someone) are also cases where this transition would be observed to occur. Again, reverting the subphase to C1 ensures that the knee will be providing resistance to flexion load, preventing its collapse and subsequent user fall.

S2 to C1 state transition **606** is observed to occur as the level walking swing phase extension knee joint motion completes and the prosthetic foot enters in contact with the ground again, registering a load on the prosthetic limb again. Detection of the loadcell sensor signal exceeding the predetermined threshold causes the phase to transition back to swing and the subphase to go back to the C1 subphase, allowing the whole cycle to start again.

Figure 7 presents the detailed state machine managing the locomotion activities, previously introduced as **404** in Figure 4. The activity state machine makes use of six distinct activities, which is well suited to cover the locomotion activities associated with a microprocessor controlled prosthetic knee using a MR actuator. However, this is not a constraint on the use of the embodiments herein disclosed and is only provided as an example of a typical inference layer control system. The number of activities, functional relationship between activities, and the nature of the conditions used to manage the transitions between activities are defined to exemplify the nature of a typical system where this would be used and can be changed without affecting the nature and spirit of the system herein described.

Standing activity state **702** defines the default behavior of the prosthetic device, as this is a commonly used activity that tends to bridge the gap between common locomotion activities observed in daily living. More specifically, standing activity is particularly well suited to bridge between locomotor and non-locomotor activities. Standing is associated with the state in which the end-user is standing still on their legs but may also include some movement that cannot be characterised as cyclical in nature. Often, ambulation associated with confined spaces, side steps, and backward steps are most efficiently classified under the Standing state. While operating in Standing, the knee joint behavior is defined as providing resistance against flexion motion, to prevent the knee from collapsing under the user's weight, and allowing extension motion with little resistance, allowing the user to easily extend the knee when required.

Sitting activity state **712** defines the state reached when the end-user is seated in a fairly still position. This state allows managing the low activity behavior observed when the user is seated on a chair or other similar object. Similar to the Standing state, the Sitting state **712** also ensures that the knee joint provides finite resistance to the flexion motion or loads, while allowing for extension with minimal resistance.

Transition into the Sitting state **712** can only be achieved from the Standing activity state **702,** by going through the sit-transfer **711.** Definition of the Sitting state **712** is established based on the general observation of this activity as performed in daily living and is measured by the prosthetic knee embedded sensor set. While not being the only way to identify that the user has reached the Sitting state, it is possible to monitor the knee joint angle for a value larger than a predetermined threshold (e.g. flexion superior to 95°), combined with a low knee joint angular velocity (e.g., less than 5°/sec), over a predetermined period of time (e.g., 5 seconds).

Sit-transfers transition **711** is a bidirectional transition making use of the fact that the sit-to-stand and stand-to sit transfer behavior can be easily integrated in the Sitting and Standing knee joint behavior. During stand-to-sit transfer, the user applies weight to the prosthetic limb while in stance phase. The knee joint providing a finite amount of resistance against flexion motion will start yielding and allows the user to gradually lower their body as the knee flexes. Once the seated position is reached and the threshold for entering the Sitting state **712** is met, state transition occurs and the knee joint behavior is updated accordingly. Sit-to-stand transfer also typically happens in stance phase, where the user leveraged the Sitting activity state knee joint behavior to start extending the knee under load, while gradually raising their center of mass. Once the knee joint is observed to present a sufficiently extended position, state transition is executed, and the knee joint reverts to the Standing activity state and the corresponding behavior.

Biking state **700** is a specialized activity state aimed at supporting the specific requirements for the knee joint when riding a bike. When operating in that activity state, the prosthetic device is configured to provide minimum knee joint resistance. This state can be entered directly from the Sitting activity state through transition **713,** or through Standing activity state and transition **701.** Transition to Biking activity **700** through the Sitting activity **712** is performed by monitoring the motion pattern of the knee joint using the embedded knee joint position sensor. As the user initiates pedalling with the knee joint presenting its usual behavior, the cyclical motion of the knee joint is observed for a number of cycles to ensure that the typical pattern observed while cycling is found, allowing the state transition to the Biking activity and the subsequent change of the knee joint behavior to enforce minimal motion resistance. A similar mechanism is used to manage the Standing to Biking transition **701,** leveraging the fact that the knee joint behavior is similar in both Sitting and Standing activities.

Biking activity state **700** can only be exited through the Biking to Standing transition 701. As there is no functional difference between the Biking and Sitting states for the user, state transitions can be simplified by allowing a single exit path along the route offering the best discriminant. Standing state **702** is entered when the knee joint is observed to meet a minimum extension target, as per registered by the embedded knee joint angle sensor, while operating in Biking activity state **700.**

Kneeling activity state **714** is a specialized activity state used for creating a stable knee joint behavior when the user is down with the prosthetic knee on the ground, leaving the prosthetic device mainly horizontal, suspended in the air between the tip of the foot and the front of the knee joint. When operating in Kneeling state, it is important to provide an appropriate level of knee joint resistance for the operation to be comfortable for the user, without being too resistive such that the end-user can still move around without being encumbered by the prosthetic device. It is also to be noted that the actual position of the knee joint in this activity state does not allow use of the loadcell sensors, as the load line going through the prosthetic foot is approximately perpendicular to the shank segment long axis, corresponding to the loadcell sensor sensitive axis. To prevent false detections arising from the low sensitivity configuration or cross talk in the loadcell itself, their measurements must be disregarded in the Kneeling activity state.

Transitions to kneeling from standing and the converse **718** are mainly executed based on the measurement of the prosthetic device shank segment's orientation using the embedded IMU sensor, along with some additional conditions specific to the biomechanical task definition. Similar transition detection conditions are also used when transitioning in kneeling from sitting activity state **716.** Upon detection of the shank segment's sagittal plane orientation being tilted by more than 60° with respect to vertical for a minimum duration of 1500 milliseconds (ms) when the knee joint is flexed by more than 5°, the activity state is transitioned from Standing **702** activity to the Kneeling activity. In the case of the Sitting **712** activity state transition to Kneeling, the condition regarding the minimum knee joint flexion angle is not enforced, as no strong need arises for ensuring that the user is really kneeling, as the current state is Sitting and the knee joint is already expected to be flexed.

In the opposite situation, detection of the prosthetic device shank segment's sagittal plane orientation being more vertical than 60° will cause the activity state to exit Kneeling state **714** back to Standing **702** activity. Kneeling activity state **714** transition back to Sitting activity state **712** is not directly supported in this embodiment of the prosthetic knee control system. From a functional standpoint, supporting this transition does not appear necessary since the end-user more or less always needs to stand-up in order to transition back into a seated position.

Inactive activity state **715** is a specialized state used to control the prosthetic device's operation when it is observed not to be in use for an extended period of time, such as at nighttime when the device is not used. It is often observed that the end-user will forget to power down the device before going to bed or when using another prosthetic device while playing sports or just resting the residual limb. In that context, this activity state can be used in order to advantageously minimize the device's power consumption and prevent battery depletion, hence providing direct assistance to the end-user in implementing basic power consumption management. Since the device is not required to be functional in this activity state, the state itself can be used to define an actuator behavior not consuming power, as well as turn off system peripherals that are not required just to keep the system in standby operation. Such an automated power management approach is considered more efficient than automatically powering off the device for the end-user, as this could generate an unexpected situation for the end-user after re-donning the device thinking this it was powered up.

Inactive activity state **715** can be directly entered from the Kneeling activity state **714** through a unidirectional transition **717.** Additionally, Inactive activity state **715** can be entered from the Standing activity state **702.** Both transitions rely on the same set of conditions and are used to cover different cases when the unused device would be left waiting while powered on. Direct Kneeling activity state transition **717** to Inactive state represent cases where the device would be lying approximately flat on the ground while not used. On the other hand, the Standing activity state transition to Inactive comprised in **719** represents a case where the device would be left standing upright while not in use. Both transitions to Inactive State represented by **717** and **719** are based on the detection that the device has been inactive for a significant period of time. For example, if the knee joint rotational velocity is observed to be lower than 8°/second and the shank segment sagittal plane's angular velocity is lower than 25°/second and the absolute value of the first derivative of the total vertical load measured by the loadcells with respect to time is lower than 50 Newtons/second for a duration of 30 seconds, the system state will transition to the Inactive activity state. These conditions represent a typical example and are not to be considered an exclusive solution. It is to be noted that since it is required to prevent transition to Inactive activity state from the Standing activity state while the device is actually worn by the end-user, the set of conditions and their threshold values must be selected to be make this transition very hard to achieve under the typical motion observed when an end-user is just trying to stand still.

Conversely, the inactive to standing activity state transition comprised in **719** must also be very sensitive to prevent a situation where the end-user would start using the device again while it is in the Inactive activity state **715.** In the case where knee joint rotational velocity is observed to be higher than 8°/second or the shank segment sagittal plane's angular velocity is higher than 25°/second or the absolute value of the first derivative of the total vertical load measured by the loadcells with respect to time is higher than 50 Newtons/second, the system state will immediately transition from Inactive activity state **715** to Standing activity state **702.** While the conditions forcing the system to transition back into Standing activity state **702** from Inactive activity state **715** are considered easy to obtain and may cause the occurrence of false positive transitions associated with environmental factors, or other similar factors not related to end-user operation of the device, the current approach is considered safer, even if the absolute power savings associated with the use of an Inactive activity state may be slightly reduced in some circumstances.

While operating in the Standing activity state **702,** transitions to the other main activities of daily living are also possible. Namely, transitions to level-ground walking **706,** Stair Descent **708** and Stairs Ascent **704** are allowed by the system. It is to be noted that this embodiment of the knee prosthetic control system does not provide explicit activity states for the incline and decline activities. For sake of simplicity and making optimal use of the prosthetic system capabilities, these activities are directly addressed through their functionally equivalent tasks of stairs descent (i.e., ramp descent) and level-ground walking (i.e., ramp ascent).

Level-ground walking activity state **706** is used to manage the end-user's forward progression when the user is ambulating on flat surfaces, but also provides support for upwards slopes, as long as the slope grade remains such that it is possible for one to walk on it without significantly affecting one's body posture or gait pattern. Level walking activity can only be entered from the Standing activity **702,** following initiation of the gait pattern by the end-user. As introduced above, transition to Level-ground Walking state **710** occurs when the user is observed to present sufficient forward progression dynamics, which is dynamically assessed by the system through the embedded sensors. Three conditions are required for the transition **710** to take place: the knee joint has to be fully extended and present a low angular velocity, indicative that the stance extension subphase has completed, an extension moment of sufficient amplitude has to be present, indicative that the user's center of mass is currently anterior to the prosthetic foot, and, finally, the prosthetic limb shank segment must show sufficient rotational speed, indicative of forward momentum. When these conditions are simultaneously met, activity state will be transitioned to Level-ground Walking state **706.**

Conversely, the activity state will be observed to transition back to Standing from Level-ground Walking in the case where a failure to maintain forward progression dynamics is observed. For example, in a situation where the user would be observed to stand on the prosthetic limb without moving for a predetermined amount of time would cause the activity state to transition back to Standing **702** through direct transition **710.** Another example of conditions observed during prosthetic device operation that would cause a direct transition back to Standing state **702** from Level-ground Walking state **706** is the case where an unexpected phase transition would occur in C1 or C2 subphase, while the KB conditions are not met. Such an unexpected transition clearly indicates an interruption of the user's forward progression and requires transition back to the Standing activity, where the basic knee joint behavior will be more appropriate to address the specifics of the locomotion task at hand.

While operating in Level-ground Walking activity state **706,** the prosthetic knee control system implements the full set of subphases previously introduced in view of optimizing the end-user's forward progression. Implementation of the timely sequence of knee joint behaviors defined by the subphases allows for creating high synergy with the end-user and makes optimal use of the momentum and commands provided through the residual limb.

From the Standing activity state **702,** it is also possible to enter the Stair Ascent activity state **704,** through the state transition **703.** This would be observed in cases where the user is standing static at the bottom of a staircase and would initiate the climb directly. Stair Ascent is the state used to support the locomotion task of climbing stairs, which requires specific knee joint behavior to be implemented. Standing to Stair Ascent state transition **703** can be executed in two different manners by the end-user, which requires the set of conditions allowing the transition to take place to be extended accordingly. In one variant of the standing to stairs ascent transition, the end-user initiates stairs climbing by moving the amputated side to the first step. This technique requires the end-user to control the prosthetic limb while flexing the hip and allowing the knee joint to flex, under the influence of the hip motion and gravity, to an angle allowing the end-user to step into the flexed knee with this one resting on the step. This variant of the transition is generally referred to the stance detection variant and positive standing to stair ascent transition is considered to have taken place when stance phase is detected while the knee is flexed over a predetermined threshold.

In the other variant of the standing to Stair Ascent transition **703,** the end-user initiates stairs climbing with his non-amputated limb first. Once the end-user steps on the first step of the staircase with their sound limb, the prosthetic limb enters Swing phase. For the transition from standing to stair ascent to take place in this variant, the embedded sensors are monitored for a large swing flexion cycle. To properly execute the transition, the user has to create swing flexion motion through use of the residual limb hip providing sufficient flexion angle allowing to clear the nose of the coming step, which normally will allow for the state transition to be considered valid.

Stair Ascent activity state **704** is used to implement the knee joint behavior required to support execution of the locomotion task once the transition conditions have been met. To support the end-user in achieving step-over-step gait while climbing the stairs case, the knee joint has to support the end-user in lifting their body weight while standing on the flexed prosthetic knee. Since the typical embodiment of a prosthetic knee joint does not use actuator technology allowing the injection of mechanical power, the knee joint behavior has to be limited to minimize resistance to knee extension under the user generated thigh motion, while providing sufficient support for preventing the knee to flex when the user is applying weight on the flexed knee joint. Additionally, knee joint behavior has to account for the fact that the knee goes through a distinct kinematics pattern than what can generally be observed in level-ground walking activity. As the actual horizontal step length while climbing a staircase is much shorter than what is normally observed in level-ground walking, and there is a need to account for the vertical displacement between the consecutive steps, the knee terminal swing flexion angle remains larger than what would be observed in other locomotion activities. Control over the terminal swing angle is implemented in Swing phase, where the knee extension motion will be stopped using the joint actuator, either allowing the end-user to step into it on the coming step or whip it out to full extension using the residual limb hip and thigh.

Stair Ascent knee j oint behavior makes uses of four subphases, which match the four knee joint behaviors required to complete step-over-step cyclical gait. While this definition is arbitrary and does not constitute a limitation in the implementation of the control system herein described, aligning the subphase definitions with the physiological aspects of the gait task simplifies the controls and feels more natural for the end-users. Observation of the non-amputated knee joint behavior in stairs ascent allows the identification of four behaviors. Following contact of the foot with the coming step and completion of the end-user's weight transfer to the stance phase limb, the knee is observed to slightly yield as the load on the knee increases. Once the knee joint has been properly loaded, stance extension will take place, allowing the end-user to lift their body weight over the supporting limb by simultaneously extending the knee and hip joints. Once the knee reaches full extension, the limb under observation transitions to Swing phase and the knee joint flexes as the hip starts flexing to bring the foot in front of the end-user, allowing the user to clear the coming step nose and riser. Finally, once the step has been cleared, the knee joint can extend and position the prosthetic foot in such a way as to support landing the foot on the ground surface, with a knee flexion angle allowing the user to step on the limb again.

As per the typical subphase knee joint behaviors introduced above, a good match exists with a subset of the subphase defined for level walking. Stance flexion behavior observed following prosthetic foot contact with the coming step directly matches the definition of the C1 subphase. Similar to the Level-ground Walking activity, knee joint stance extension is supported by the C2 subphase. Swing flexion is by definition covered under the S1 subphase, while Swing extension is covered by S2. Based on these subphases, cyclical step-over-step stairs ascent is achieved through the C1-C2-S1-S2 subphases sequence.

Stairs ascent to standing state transition is also included in the **703** state transition, which is in fact represented as a bidirectional transition. This state transition is mainly driven as the failure to meet the conditions for the standing to stair ascent transition or failing at continuing the task in a timely manner. For example, one condition causing the direct transition back to Standing state while operating in Stair Ascent state is from the detection of Stance phase while the knee is extended beyond what is considered normal for performing step-over-step stairs ascent on typical staircases. This type of event would normally be observed when the end-user has completed the staircase climb and is now stepping on the prosthetic limb on the top floor, or the end-user has interrupted the step-over-step climb to place the prosthetic limb on the same step where the sound limb is. Additionally, this immediate transition is also valuable in cases where the end-user is struggling to control the knee joint angle in swing extension and ends up with an extended knee, which will not allow them to complete the step ascent. In such case, reverting the knee behavior to standing activity allows for greater flexibility to the end-user for stepping back and starting the stair ascent again, while providing higher safety for the end-user.

In a similar fashion, failure of the end-user to fully extend the knee before entering Swing phase while operating in stairs ascent activity will cause an immediate transition back to standing activity. Failure to complete the Stance phase extension motion negatively affects the end-user's capacity to power swing flexion, which could lead in toe stubbing or landing on an excessively flexed knee joint, which is not desirable when operating on a staircase. Another scenario where this could be observed is when the end-user is resting the prosthetic limb on a chair or fairly high obstacle, without the intent of climbing it. In such as case, immediately reverting to Standing mode when Swing phase is detected allows for a more natural transition and provides a better correlation to the user's intent. Finally, transition back to Standing activity state is also enforced when the end-user is observed to stand on the knee for a predetermined period of time without triggering a new subphase. This use-case matches the scenario where the user has stopped, in which case there is no need for the state to remain in Stair Ascent, the end-user effectively being in Standing activity.

Level-ground Walking to Stair Ascent state transition **705** is also possible in the current embodiment of the prosthetic device control system. This transition is implemented as a one-way transition, since the opposite transition is not considered beneficial in view of the typical locomotion pattern observed in amputees. Transition from Stair Ascent to level-ground walking can be easily implemented with an intermediate transition through standing, which does not affect the system's performance and is not perceived as a functional limitation. On the other hand, requiring the end-user to transition back to Standing to transition to Stair Ascent would cause an interruption in their forward progression, which is perceived as a functional limitation. Dynamic transition from Level-ground Walking to Stair Ascent **705** leverages the similarity between the standing activity and level-ground walking knee joint behavior, which allows using the same transition condition for transitioning with sound limb first entering the staircase. By first stepping on the first step of the staircase with the sound limb, the user can modify their hip motion pattern to increase the swing flexion amplitude, both creating the additional clearance for the prosthetic foot to clear the coming step nose and allowing the detection of the dynamic transition by the control system.

In some embodiments, the knee is flexed at initial heel contact, and is locked and easily extended with extension knee torque when the user makes his way over the limb. This advantageously improves gait symmetry as the user of ascends the ramp. The initial heel contact knee flexion may increase as the ramp slope increases, to make ambulating easier for the user during ramp ascend.

Again, from the Standing activity state **702,** it is possible to transition to the stair descent activity state **708,** through state transition **709.** Transition from Standing state is representative of a scenario where the end-user is standing static at the top of a staircase or a slope, and initiates progression through the obstacle. Stair Descent state **708** is then the state used to implement the specific knee joint behavior required for supporting the end-user's progression through the obstacle. As it is rather cumbersome to get the knee joint to yield while standing at the top of a staircase, Stair Descent is typically initiated with the prosthetic limb first. On the other hand, ramp descent offers more flexibility as the need for the prosthetic knee to yield allowing the sound limb to be placed on the lower ground surface is not as stringent. Additionally, for lower grade slopes, it is also typical for the end-user to enter the slope through the level-ground walking activity and only revert to using the Stair Descent function once fully entered in the terrain, or when the grade reaches a level where it is no longer efficient or comfortable to keep operating using the level-ground walking activity.

Standing to stairs descent transition **709** occurs when a set of specific conditions are met that confirms that the general end-user progression matches the general pattern associated with a highly energy dissipative task. As the user steps in the knee joint still operating in Standing activity, knee flexion will result as the user transfers their weight to the prosthetic limb. Additionally, to the flexion motion, high flexion moment should be observed as the end-user keeps their body weight posterior to the supporting limb and knee joint. Finally, end-user forward progression component created by the combined knee and hip flexion, coupled to the prosthetic foot rolling over the nose of the step, should generate sufficient angular rotation motion for both the thigh and shank segment. When these conditions are met, the activity state immediately transitions to Stair Descent.

From a biomechanical standpoint, operation of the knee joint in Stair Descent activity state **708** requires the implementation of specific stance phase behavior to address the need to dissipate a significant amount of energy as the end-user body weight is lowered from one step to the next, as well as yielding the knee joint in a controlled manner, allowing the end-user to maintain balance and proper forward progression. Addressing the gravity generated loads and increased leverage caused by the increasing knee flexion as the stance phase evolves requires providing additional yielding resistance in Stair Descent with respect to what would generally be observed in other locomotion activities. However, attention must be directed at not providing too much resistance, as a risk of locking the knee joint and causing the end-user to vault over the locked knee exists which could cause a stumble or fall to take place. Nominal knee joint yielding resistance level shall account for the user's body weight and gait style but use of a continuous motion control scheme is also desirable as other factors can dynamically affect the stairs descent gait task execution, leading to insufficient resistance. For example, use of static resistance level in stair descent would lead to faster yielding when the user has to carry an object, which effectively instantaneously modifies its body weight. Additionally, one has to account for ambulation over varying step height or staircase pitch, which in turns changes the kinematic profile of the knee joint during the gait task execution, which can result in varying distance between the end-user's body weight and the knee joint, effectively changing the end-user leverage.

Stair Descent locomotion task can be broken down in a limited number of subphases, once again using the basic subphase set and definition established for level-ground walking. Stair Descent gait at knee joint level is composed of three specific components. In Stance phase, the knee joint has to control the yielding motion and allow for progressive lowering of the end-user's body weight. When Stance phase completes, the prosthetic foot loses contact with the step surface and knee joint flexion happens, allowing to further clear the step and preventing the occurrence of toe-stubbing. Requirements for swing flexion knee motion can be mitigated by the user gait style. When the end-user positions the foot very far out on the step thread, leaving a large part of the prosthetic forefoot not in contact with the step surface, the need for swing flexion to prevent toe-stubbing as the end-user keep progressing forward is not as stringent. Nevertheless, since the form factor of the steps in staircase can be highly variable and keeping the forefoot supported on the step while ambulating down staircases greatly improves the end-user's balance, it is important to consider the need to implement swing flexion as part of the gait task modeling. Finally, once swing flexion motion has completed and allowed to clear the prosthetic foot from the step, swing extension can take place, positioning the foot and the lower limb in preparation for the upcoming foot contact with the next step.

In some embodiments, during ramp descent, the knee is kept flexed, by no more than 15°, before initial heel contact with the ground to allow the user to flex the knee with ease during early Stance phase. Usually, the knee is fully extended when entering Stance Phase and if the user does not start the Stance phase with a flexion torque, the knee will keep being extended causing discomfort to the user and possibly unstable behavior. Having a slightly flexed knee during ramp and stair descend addresses this problem and is easier for the user. The initial heel contact knee flexion may increase as the decline increases, to make ambulating easier for the user during ramp descend.

In terms of subphases, these 3 main components of the stair descent gait can be described using the Level-ground Walking subphase matching the desired behavior. Knee joint stance flexion under load matches the behavior associated with C1 subphase. Swing flexion is once again supported through the S1 subphase and swing extension by the S2 subphase. Cyclical step-over-step stair descent gait is then described by the subphase sequence of C1-S1-S2.

Stair Descent activity state **708** can also be reached from transitioning directly from Level-ground Walking, as per illustrated by transition **707** in Figure 7. Once again, due to the similarity between certain aspects of standing activity and level-ground walking activity, dynamically transitioning between Level-ground Walking and Stair Descent is more or less functionally equivalent to transitioning between Standing activity and Stair Descent activity. Since every level-ground walking step typically meets the functional requirement for executing the standing to level-ground walking step, using the same condition as in the standing to stair descent transition **709** allows implementing the level-ground walking to stair descent transition **707.** As the end-user ambulates towards the staircase and places the prosthetic limb on the first step to continue progressing through the obstacle, the prosthetic limb's mechanical configuration will allow the end-user to apply a higher load than usual on the knee joint, creating additional knee joint flexion over what is normally observed (e.g., in level-ground walking). Additionally, due to the higher end-user leverage on the knee joint, high flexion peak torque will take place and can be monitored by the embedded control system. Finally, forward rotation of the lower limb as the end-user's center of mass progresses over the prosthetic foot will occur and can be monitored by the inertial measurement unit connected to the shank segment. When all three variables are observed to meet their predetermined threshold, state transition **707** will be executed and knee prosthetic current activity state will become stairs descent.

As introduced above for the dynamic transition from level-ground walking to stairs ascent **705,** dynamic transition from level-ground walking to stair descent **707** is also a unidirectional transition. Stair descent to level-ground walking explicit transition management is not required as the conditions managing the stair descent to standing activity transition under **709** are such that when the end-user is stepping on the level-ground surface at the bottom of the staircase, the set of conditions to remain in stair descent activity state will not be met, causing the system to immediately transition to Standing activity state **702.** As per mentioned above, since all typical cyclical level-ground walking steps meet the conditions to transition to level-ground walking activity state, the system will fall back to the desired state after a quick transition through the standing activity state, which will not cause a reduction in perceived system performance.

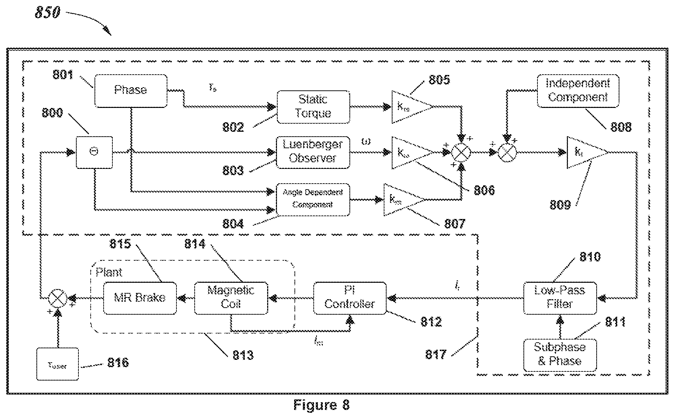

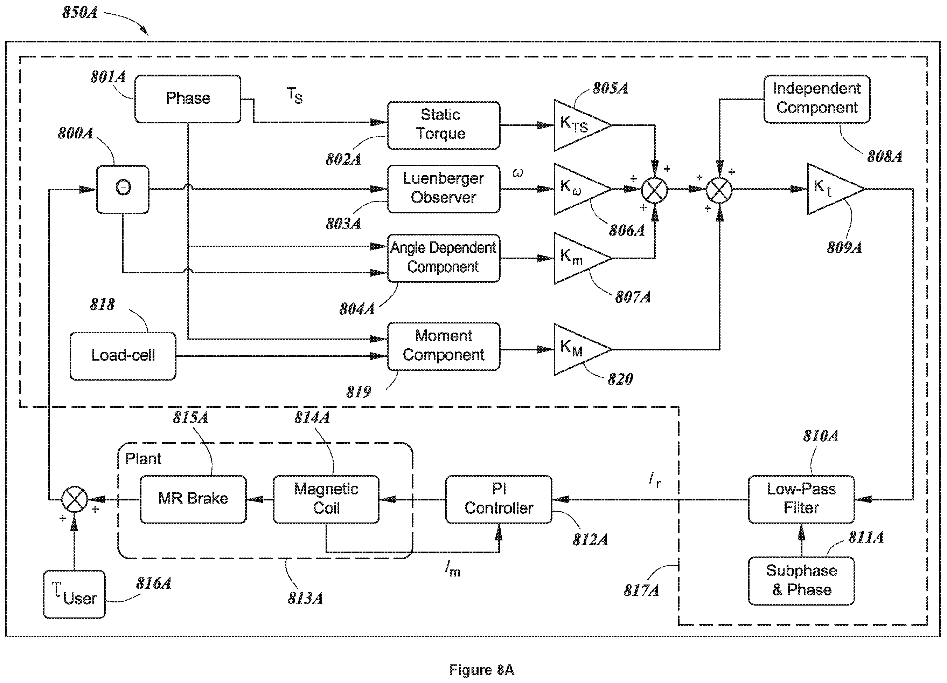

Figure 8 presents an example of the high-level knee joint actuator control scheme block diagram, or outer loop control scheme **850.** The control loop **850** aims at generating the proper knee joint behavior based on the objectives defined for each subphase, in each activity, such that predefined gait support strategies are implemented in real-time, as the end-user is operating the knee prosthetic. In one non-limitative embodiment, the outer loop control scheme **850** is implemented as a combination of pure velocity control and some additional heuristic components, which are herein referred to under the general nomenclature of velocity controller **817.** The outer loop control scheme **850** is implemented as a digital controller in the microprocessor **207.** The MR actuator itself is represented by the plant **813,** which is further broken down in magnetic coil **814** and MR brake **815** blocks. While the magnetic coil **814** is normally directly embedded in the MR brake **815,** representing them separately simplifies the system modeling, even if it is less representative of the physical embodiment. A knee prosthetic as a whole is also subject to interaction with the user and the environment, which is represented as a perturbation torque **816.** The combination of the perturbation torque **816** with the knee prosthetic and actuator-generated braking torque are the target of the control scheme **850** herein presented, as it represents the overall device response when used, which is herein represented as a resulting knee position **800.** The resulting knee joint position is measured by position sensor **206** and is fed back to the outer loop control scheme.

MR brake **815** provides a controlled amount of resistance to motion based on the magnetic field strength going through the MR fluid. Magnetic field is induced by the magnetic coil **814** dependent on the amount of current circulating in the magnetic coil **814.** While MR brake **815** resistance is generated in open loop, magnetic coil is controlled in closed loop, through regulation of the current, which is performed through a digital Proportional-Integral (PI) controller **812,** implemented in either the device embedded microcontroller or any other digital processing platform available on the device. PI controller **812** compares the current measured in the magnetic coil *Iₘ* with the current set-point requested from the outer loop controller *Iᵣ* to determine how to adjust the command provided to the coil driver circuit in view of ensuring that the measured current *Iₘ* matches the requested current *Iᵣ.* Adjustment to the command signal to coil driver circuit is directly performed by the PI controller **812,** where the gains are adjusted to ensure that the correct dynamic response will be obtained from the current controller when the set-point is modified by the outer loop controller. In one non-limitative embodiment, the coil driver circuitry is implemented using a pulse-width modulation scheme, where the duty cycle during which the magnetic coil is connected to the battery supply is proportionally adjusted based on the amplitude of the current command issued by the PI controller **812,** leading to an increased average current in the magnetic coil when the current command is increased.

The resulting combination of the MR actuator's resistance to motion, user interaction and perturbation torques from the user and the environment result in the prosthetic knee joint's instantaneous position Θ **800**. Actuator position data **800** is fed into a Luenberger Observer **803** to generate an actuator rotational velocity estimate ω, which is then fed through the velocity gain K_{ω} **806.** The velocity term of the control loop is the main continuous control element, as a strong correlation exists between actuator velocity and the required actuator resistance level required for the user to successfully complete the locomotion task undertaken. To support the specific actuator behaviors, three discrete terms are added to the control loop and are executed based on specific conditions associated with the device usage (i.e., gait phases and subphases, **801** and **812**).

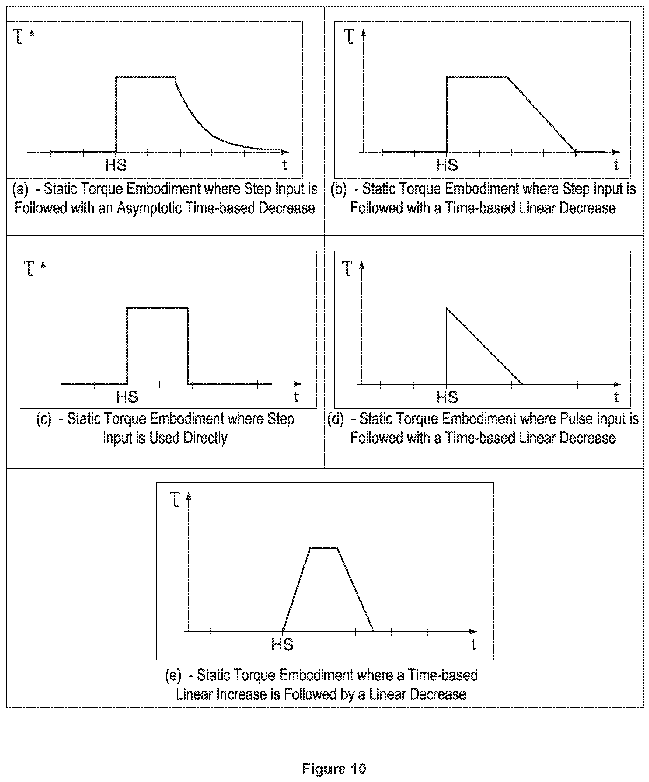

The Static Torque **802** block consists of a feedforward term that is fed through a static torque gain K_{TS} **805** and forces a minimum actuator resistive torque when the actuator velocity is null or below a certain threshold, while the knee prosthetic enters the Stance phase following the user's weight transfer over the prosthetic foot. While the characteristics of the static torque profile can be varied based on the exact nature of the locomotion task, a general characteristic where amplitude is observed to decay in time following triggering is considered optimum as it provides a better loading response to the user's weight without preventing the user from quickly moving the knee if desired. In a situation where the actuator velocity would be sufficiently high, the velocity arm of the controller (Luenberger Observer **803** and velocity gain **806**) would provide enough responsiveness and there is no hard need to add the contribution of the static torque **802** component. Static torque triggering is driven by the Phase detection engine **801,** which was previously introduced in Figure 5. Figure 10 presents examples of different torque versus time profiles used to generate the static torque input to the control system. In these plots, the torque setpoint is presented on the vertical axis, while the horizontal axis presents the time. Occurrence of the contact between the prosthetic foot and the ground surface is represented on the horizontal axis with the 'HS' designator (i.e., Heel Strike). Occurrence of the foot-ground contact is herein indicated by the phase detection and management state machine previously introduced. Implementing the general pattern required to obtain the desired function the torque profiles typically present a sharp rise in torque level following the occurrence of the prosthetic foot contact with the ground surface, followed by varying time-based profiles. Figure 10(a) presents a static torque profile where the high torque value is provided upon occurrence of the foot-ground contact and maintained for a fixed duration before being brought back to null value using an asymptotic profile. Such static torque characteristic provides a strong knee joint response to the loading generated by the user, favoring good proprioception by the user, while the static component removal is initially very fast, before tapering in a slow reduction rate due to the asymptotic trend. This type of profile advantageously provides a highly stable dynamic knee joint response, while still providing high flexibility due to the fast removal rate.

Figure 10(b) presents a static torque profile where the high torque value is provided upon occurrence of the foot-ground contact and maintained for a fixed duration before being brought back to null value using linear profile. Similar to what was observed for Figure 10(a), such a static torque characteristic provides a strong knee joint response to the loading generated by the user, favouring good proprioception and perceived stability and safety, while a more gradual static component removal is used. This type of profile then advantageously favors safety and stability by providing both high loading response and consistent support during the static torque removal period. Such a profile is found beneficial for less dynamic users who do not have a need for very fast transitions between Swing and Stance phases, which then obtain increased support and stability with such profile.

Figure 10(c) presents a static torque profile where the high torque value is provided upon occurrence of the foot-ground contact and maintained for a fixed duration before being removed instantaneously. Again, a sharp rise of the static torque characteristic provides a strong knee joint response to the loading generated by the user, which, in this case, is coupled with a fast removal. This profile then combines the benefits of the positive loading response, which optimizes the flexibility for end-users capable of rapid dynamic phase transitions as part of their daily activities. Figure 10(d) pushes this concept further by reducing the high torque value component of the static torque profile to a minimum duration, followed by a linear removal of the static torque contribution. Such a profile then relies on the end-user to properly control the knee joint during the loading component of the swing to stance phase transition, which is beneficial for strong and highly dynamic users showing good proprioception that can easily operate the prosthetic knee with minimal support in a variety of situations.

Figure 10(e) presents a static torque profile where the high torque value is gradually provided upon occurrence of the foot-ground contact, maintained for a fixed duration before being gradually removed. Gradual increase of the static torque setpoint during the prosthetic device loading stage can be beneficial for use cases where a smoother knee response is required, either to allow for higher energy dissipation at foot-ground contact, provide some level of shock absorption, or allow the end-user to control the initial knee yielding before getting support, such as could be observed in ramp descent initiation, for example. Again, removal of the static torque contribution linearly provides a consistent response, which positively contributes to the user confidence in the device. It is to be noted that these examples are not to be considered limitative and can also benefit from dynamically configuring the exact parameters defining them, such as static torque amplitude, ramp-up or ramp-down duration and trend, etc.

Phase detection engine **801** provided information is also fed to the Angle Dependent Component block **804,** along with the actuator position **800** information. The Angle Dependent Component **804** (e.g., set point generation, transfer function) is a feedforward term that is fed through a gain Kₘ **807** applied during Stance phase but where the characteristic of the contribution of this arm of the controller is scaled according to the actuator position **800.** Typically, but not to be considered as a limitative embodiment, this feedforward contribution is shaped like a saw tooth waveform, providing an increasing contribution over approximately the first half of the actuator motion range, before decreasing back to its original level over the second half of the actuator motion range.

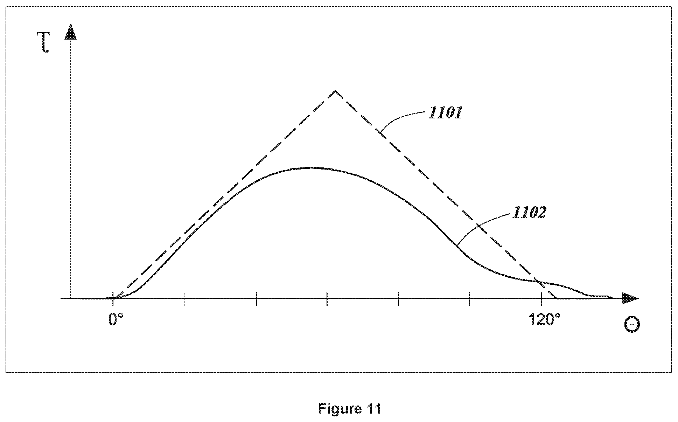

Figure 11 presents some examples of possible Angle Dependent Component torque setpoint characteristics, such as the sawtooth profile introduced above. As mentioned, the angle dependent component (e.g., set point generation, transfer function) provides a torque set-point for the control system, where the value of the set-point is dependent on the knee joint angle. Figure 11 then plots the torque set-point on the vertical axis, while the knee joint position is represented on the horizontal axis. Various torque set-point profiles can be defined, such as presented by the sawtooth profile **1101** and the smooth profile **1102.** While no specific limitations exist regarding the general shape of the profile, some benefits arise from a profile providing increased torque set-point over the beginning of the flexion motion range, where biomechanical data indicates that most of the functional knee joint loading will occur.

The Angle Dependent Component then provides a base level actuator resistance torque which is scaled up depending on actuator angle when the prosthetic knee is operating in stance phase. This is observed to reduce the actuator performance dependency on the flexion-extension actuator velocity, which allows for more consistent support of the user weight with increasing flexion angles and during motion direction changes, where velocity goes through a null point and where the user could be left without actuator resistance while loading the actuator. Additionally, the Angle Dependent Component **804** feedforward term can be defined in such as way as to compensate for the increased actuator loading from the user as the angle increases, which causes an increase of the distance between the user upper body weight and the knee rotation axis.

Finally, the Independent Component **808** is added to the three other arms of the controller. While the Independent Component **808** is a feedforward term which is fed through a gain kₜ **809** like the static torque **802** or the Angle Dependent Component **804,** this one does not have an explicit dependency to a gait control parameter like the actuator angle **800** or the Phase **801** but is more heuristic in nature and accounts for specific actuator behaviors that are not properly accounted for by the three main branches of the controller structure. Typically, these behaviors are driven from specific user need and are not fundamentally part of physiological gait *per se.* For example, when maintaining the knee in full extension in swing phase is desired, the independent term is used to make the knee resistance increase to a level where the knee will remain in full extension, without any velocity term contribution. Similarly, it is found desirable to increase the actuator resistance prior to foot strike to mitigate any delays observed in ramping up the actuator resistance and user perceived knee buckling at initial loading, which is performed using the independent component **808,** as velocity is also null in this case and the knee is operating in swing phase. One other example where the independent component **808** is used to directly affect the actuator behavior is in stair ascent foot placement management. As the knee flexion angle at foot strike is much higher in stairs ascent then in level ground or ramps ambulation, there is a need to stop the swing extension cycle to allow the user to step on the upcoming step, when climbing stairs step-over-step. When the appropriate actuator angle is reached, the high-level stairs ascent management will generate a short increase in actuator resistance to stop the extension motion, allowing the user to step on the flexed knee or kick-it to full extension in the case where transition back to walking is required.

The resulting sum of the four different types of contribution to the controller effort is then fed to the low-pass filter **810** block. The low-pass filter **810** characteristics are dynamically adjusted as a function of the Phase and Subphase information **811,** provided by the phase state machine of Figure 5 and the subphase state machine of Figure 6, allowing to achieve optimal filtering and minimal latency on actuator reaction. For example, Swing phase does not show high-bandwidth set-point changes and a uniform behavior. It is then addressed with a single set of filter parameters. On the other hand, Stance phase in most locomotion activities is observed to present high-bandwidth set-point changes due to the user's interaction with the environment. Multiple parameter sets are then used in stance phase to ensure obtaining the optimal filter response time for all various situations. One particular low-pass filter embodiment is found to be particularly suited for the task at hand and is the 2^{nd} order Butterworth filter. While not being a limitative embodiment, this filter is known to present an optimal response in the pass band with minimum latency, which is of value in the context of a real-time system.

Use of the low-pass filter on the setpoint allows getting rid of the high frequency components and smooths out the input signal for control purposes. High-frequency components are typically generated by sensor noise, which are propagated and amplified through the different control scheme components. The high-frequency content also includes discontinuities found in the static torque **802,** Angle Dependent Component **804** constitutive terms of the setpoint signal, as well as the independent component **808.** While specific considerations can be taken to minimize the discontinuous parts generated by the independent contributors to the control signal, some are unavoidable in certain scenarios and use of the low-pass filter uniformizes the control signal as a whole, without requiring a heuristic approach. Finally, the Luenberger-observer **803** also has a contribution to the high-frequency components since it essentially acts as a controller on the predicted and measured actuator positions with a fairly high gain. Attenuation of the aforementioned high-frequency noise and discontinuities removes audible noise generated in actuator, contributes to attenuating possible chatter in the control loop, and provides a smoother experience to the user. Specific attention is required in compromising between having too small of a bandwidth and the delay of the filter, but the chosen design allows for minimal ringing artifacts in both the pass and stop-band, whilst still allowing for a steep drop-off.

Implementation of the subphase behaviors previously introduced to support locomotion activities using a typical microprocessor-controlled MR actuated knee prosthetic requires the control system to be centered around velocity control. This need arises both from the fact that the knee joint actuator is mainly used to dissipate energy provided to the system as external perturbations and the fact that it is not really practical to define position trajectories to support the knee joint resistance generation pattern, as the nature of the lower-limb tasks does not involve use of consistent position set-points. In that context, the use of velocity control appears more sustainable from an application perspective. However, implementation of closed-loop velocity control requires adequate velocity sensing, which is known to be difficult to achieve through sensing technology, when accounting for the weight, power consumption, volume and cost limitations associated with the integration of the technology in a lower-limb prosthetic device.

Velocity sensing in rotating machinery is known to present challenges. Few technologies are known to provide direct velocity quantification and translation into a signal that can be easily digitized. Traditional approaches normally rely on using active electrodynamic elements, such as AC or DC tachometers, which provide an output voltage proportional with the measure rotational velocity. However, most of these rely on commutation and are known to generate a choppy signal as a result. Moreover, complexity in implementing a tachometer in a highly integrated device presents a challenge. Other approaches make use of the possibilities related to the use of incremental inductive or magnetic sensors, where fixed angular increments can be counted using magnets or ring gears placed in front of a sensor head, allowing to count the elapsed time between two successive feature appearances. Another approach relies on the use of the doppler effect to measure the frequency shift between a transmitted and a reflected signal. While known to provide a high level of accuracy, these techniques are typically found to be more practical when measuring translational velocity and not rotational ones.

In view of these difficulties, designers often rely on differentiating the signal associated with displacement of angular position sensors. While this approach is known to amplify the noise relative to the usable signal, it often provides an acceptable compromise and allows for more optimal system integration, by making more with a limited set of system components. Multiple approaches have been developed to control the noise amplification when using a differentiated position signal to generate a velocity signal. Basic approaches will rely on filtering to remove the high frequency content introduced from the first order differentiation. However, due to the need to minimize delays introduced in the sampling chain to maintain high performance closed-loop velocity control and the generally high-bandwidth required by advanced lower-limb prosthetic control system, this approach is often observed to be limited in performance.

Another approach known to allow for more refined noise reduction in differentiated velocity signals uses a state observer to estimate the state of the system instead of simply filtering the raw velocity signal. While a simple feedforward filter applies a fixed transfer function (e.g. setpoint generation) to a given input signal without any feedback on the outcome of the filtering process being considered, a state observer provides an estimate of the internal state of a given system, from measurements of the input and output of the system. This approach is particularly well suited when the internal state of the system cannot be measured by direct observation, while indirect effects of the internal state can be observed by way of the system outputs. Combination of the measured output with a basic model of the system and input signals enables estimating the internal state with high accuracy and faster convergence than while using a filtering approach. This particular strategy has been used in the embodiment herein described and takes the form of a Luenberger observer **803.**

Figure 8A presents another example of a high-level knee joint actuator control scheme block diagram, or outer loop control scheme **850A.** Some of the features of outer loop control scheme **850A** are similar to features of the outer loop control scheme **850** in FIG. 8. Thus, reference numerals used to designate the various features or components of the outer loop control scheme **850A** are identical to those used for identifying the corresponding features of components of outer loop control scheme **850** in FIG. 8, except that an "A" has been added to the numerical identifier. Therefore, the structure and description for the various features of the outer loop control scheme **850** and how it's operated in FIG. 8 are understood to also apply to the corresponding features of the outer loop control scheme **850A** in FIG. 8A, except as described below.

In one non-limitative embodiment, a moment component **819** that is dependent on phase **801A,** and the moment measured by a load-cell **818** is fed through the moment gain K_{M} **820.** The moment component **819** contribution is scaled according to the load-cell measurements during Stance Phase and added to the other arms of the controller. The addition of the moment component **819** has the benefit of allowing the other control gains (e.g., K_{TS} **805A,** K_{w} **806A,** Kₘ **807A**) to be decreased to improve the performance of the prosthesis. When the velocity gain **806A** contributes too much knee resistance the knee will intermittently stall due to too much knee resistance and create chatter. The moment component's **819** contribution can however reduce the velocity dependency and reduce the likelihood of chatter occurring. Another benefit of the moment component **819** is that it can also reduce the static torque component gain K_{TS} **805A** and the angular dependent component gain Kₘ **807A.** The static torque **802A** contribution can be increased with increased flexion moment provided by the user, which can either be that the user is heavier, or the knee is loaded more. The Angle Dependent Component's **804A** contribution can be increased in the same manner for users that do need more support when the knee is flexed and reduces the likelihood of lighter users unable to bend the knee further. The static torque component gain **805A** and the angular dependent component gain K_{TS} **807A** can also be reduced when the user's flexion moment is less than the actuator resistance or when the extension moment indicates the user wants to extend the knee.

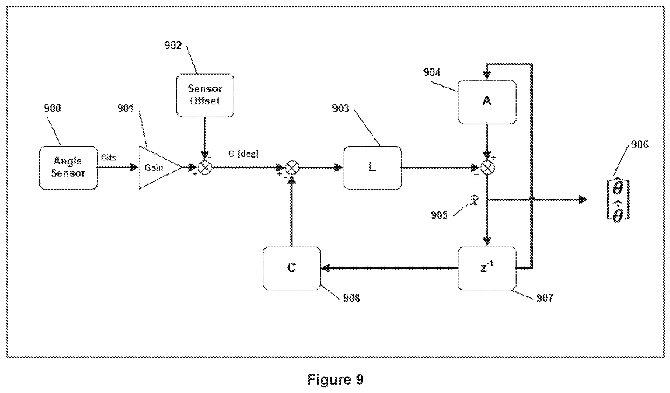

Figure 9 presents a block diagram representative of a Luenberger observer to estimate velocity in a prosthetic knee joint where only position sensing is provided. Angle sensor **900** is embedded in the prosthetic knee device and integrated in the sensing system as presented in Figure 2. Angle sensor **900** can be used on a variety of technologies commercially available, for example, such as magnetic encoders, resolvers, optical encoders, potentiometers or other similar technology allowing to provide a direct quantification of the knee joint angular position, in an absolute or relative manner. Angle sensor's **900** output signal is first preprocessed to correct the data output representation from the native bits format into engineering format, such as degrees or rads. The data representation change is implemented through the use of a gain **901,** as the relationship between the two data representation formats is purely linear. In the case where an absolute sensor is used or there is a need to align the sensor reference system with the knee joint reference system, Sensor offset **902** term is added to the converted sensor output. It is to be noted that if the system operation purely relies on the estimated velocity, there is no need to correct the sensor offset or alignment of the sensor and knee joint referential, as the velocity only relies on the differentiation of the signal and will itself remove all fixed positional reference. Preprocessed angle sensor's **900** signal now expressed in degrees and with correct offset value can then be fed to Luenberger observer **903.**

The state of a linear, time-invariant physical discrete-time system is assumed to satisfy $x \left(k+1\right) = Ax \left(k\right) + Bu \left(k\right)$ (1) $y \left(k\right) = Cx \left(k\right) + Du \left(k\right)$ (2) which indicate that the plant current outputs *y*(*k*) and its future state *x*(*k* + 1) are both determined solely by its current states *x*(*k*) and the current inputs *u*(*k*)*.* The observer model of this physical system is then derived from the above equations, where additional terms can be included to ensure that, upon receiving successive measured values of the plant's inputs *u*(*k*) and outputs *y*(*k*)*,* the model state vector converges to that of the plant. In particular, the output of the observer may be subtracted from the output of the plant and then multiplied by a matrix L, forming the Luenberger observer.

The Luenberger observer can be expressed in general form through its discrete time state space equations: $\hat{x} \left(n+1\right) = A \hat{x} \left(n\right) + Bu \left(n\right) + L \left(y\left(n\right)-\hat{y}\left(n\right)\right)$ (3) $\hat{y} \left(n\right) = C \hat{x} \left(n\right) + Du \left(n\right)$ (4)
where:
*x̂* is the observer state;
*A* is the state transition matrix and describes the dynamics of the system;
*B* is the input matrix;
*u* is the system input matrix;
*y* is the system output matrix;
*ŷ* is the observer output matrix;
C is the output matrix; and
D is the feedforward matrix.

In the particular case of a knee prosthetic integrating an angle sensor 900, the system state vector is defined as: $\hat{x} = \left[\begin{matrix}θ \\ \dot{θ}\end{matrix}\right]$ (5)
and the constituent matrices can be simplified down to: $A = \left[\begin{matrix}1 & Δ t \\ 0 & 1\end{matrix}\right]$ (6) $B = \left[\begin{matrix}0 \\ 0\end{matrix}\right]$ (7) $C = \left[1 0\right]$ (8)
and $D = 0$ (9)

In the described embodiment the system is modelled as a constant acceleration model, in other words it assumes constant acceleration over the sample time and corrects for any errors made by this assumption by looking at the difference between the measured and modelled position at the next computation-iteration of the observer. This can effectively be considered as a controller, with the aforementioned difference (L(y(n)-ŷ(n))) as the input error.

Since the objective in this implementation is to get a signal representative of the angular velocity in a manner suited for an embedded environment, a two-state system, angle and its angular velocity are used. Further states can be added to be observed, but their relation to the other states must be described. Exacter models would provide more accurate observations. In essence the described relation between the position and angular velocity here is $\frac{dθ}{dt} = \dot{θ}$, in other words the time derivative of the angular position is the angular velocity; stating this essential relationship is the entire system model dynamics in continuous time $\left[\begin{matrix}0 & 1 \\ 0 & 0\end{matrix}\right]$. Discretization by ${A}_{d} = {e}^{\left[\begin{matrix}0 & 1 \\ 0 & 0\end{matrix}\right] Δ t}$, then gives the computed matrix describing the dynamics of the model.

Time invariant gains for the observer then remain to be computed, which can be achieved through several different methods, such as pole placement, which involves finding robust solutions to the so called Riccati-equations, derived per system. Care must be taken such that gains are used, that do not result in an unstable system. The observer is called asymptotically stable if the observer error *e(k) = x̂*(*k*) *- x*(*k*) converges to zero when *k* → ∞ . For a Luenberger observer, the observer error satisfies *e*(*k* + 1) *=* (*A - LC*)*e*(*k*)*.* The Luenberger observer for this discrete-time system is therefore asymptotically stable when the matrix *A - LC* has all the eigenvalues inside the unit circle. $\begin{matrix}\left[\begin{matrix}\hat{θ} \left(n+1\right) \\ \hat{\dot{θ}} \left(n+1\right)\end{matrix}\right] = \left[\begin{matrix}0 & Δ t \\ 0 & 0\end{matrix}\right] \left[\begin{matrix}\hat{θ} \left(n\right) \\ \hat{\dot{θ}} \left(n\right)\end{matrix}\right] + \left[\begin{matrix}0.32 \\ 25.5\end{matrix}\right] \left(y\left(n\right)-\hat{y}\left(n\right)\right) & \left(10\right) \\ y \left(n\right) = \left[1 0\right] \left[\begin{matrix}\hat{θ} \left(n\right) \\ \hat{\dot{θ}} \left(n\right)\end{matrix}\right] & \left(11\right)\end{matrix}$

The gains of the observer **903** as shown in Figure 9 are used to counteract the violations of the constant acceleration assumption by being multiplied by the difference coming from the measured position (given and derived by **900, 901** and **902**) and the output from the model, coming from **908**. **908** is the output matrix, transforming the states into an output of the model, which for this implementation is the first state, with no scaling factor. The matrix describing the dynamics of the model **904** has the states of the previous computation-iteration as an input and computes the change of the states over the duration of the sample time. The states **905** are as mentioned for this implementation, the angular position of the knee joint and its derivative, the angular velocity of this same joint. The newly computed states are then taken directly as an output of this entire scheme in **906** the newly predicted first state of the angular position is neglected and not used, whereas the second state of predicted angular velocity is used as an estimation of speed and taken further into the control system. Finally, the frequency domain variable of **907** describes a delay of a single computation iteration of the observer. Since it is not possible to predict the future by using information from the future, information from the past has to be used to make these predictions.

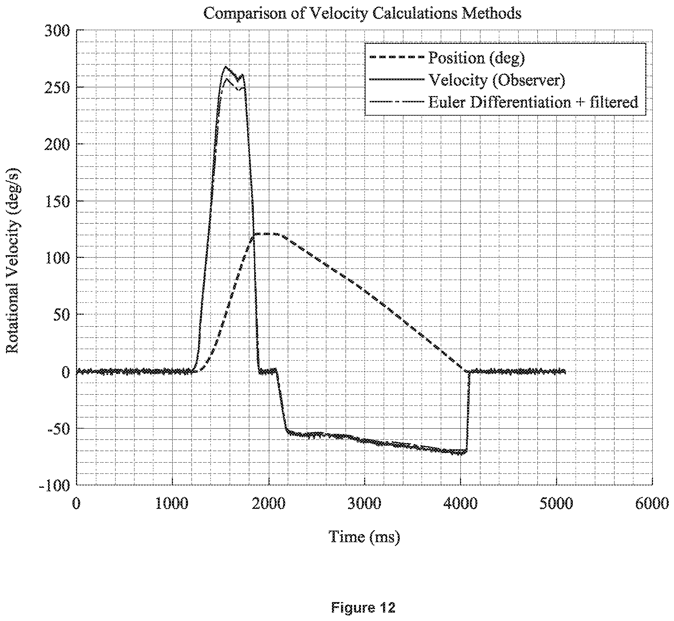

Figure 12 presents a comparison between different methods of calculating velocity. The traditional method uses Euler differentiation, taking the difference between position samples and then dividing over the time difference with a second order Butterworth low-pass filter. The method used in the disclosed embodiment uses a Luenberger observer. The measured angle is shown as well. The movement shown resembles a flexion movement from maximum flexion to full extension. Figure 12 shows that the described method follows the same behavior as the traditional way, with a smaller delay on the velocity, in the range of 10 to 15 milliseconds, although with a slightly higher noise-level.

While certain embodiments of the inventions have been described, these embodiments have been presented by way of example only and are not intended to limit the scope of the disclosure. Indeed, the novel methods and systems described herein may be embodied in a variety of other forms. Furthermore, various omissions, substitutions and changes in the systems and methods described herein may be made without departing from the disclosure. The accompanying claims and their equivalents are intended to cover such forms or modifications as would fall within the scope of the disclosure. Accordingly, the scope of the present inventions is defined only by reference to the appended claims.

Features, materials, characteristics, or groups described in conjunction with a particular aspect, embodiment, or example are to be understood to be applicable to any other aspect, embodiment or example described in this section or elsewhere in this specification unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The protection is not restricted to the details of any foregoing embodiments. The protection extends to any novel one, or any novel combination, of the features disclosed in this specification (including any accompanying claims, abstract and drawings), or to any novel one, or any novel combination, of the steps of any method or process so disclosed.

Furthermore, certain features that are described in this disclosure in the context of separate implementations can also be implemented in combination in a single implementation. Conversely, various features that are described in the context of a single implementation can also be implemented in multiple implementations separately or in any suitable sub-combination. Moreover, although features may be described above as acting in certain combinations, one or more features from a claimed combination can, in some cases, be excised from the combination, and the combination may be claimed as a sub-combination or variation of a sub-combination.

Moreover, while operations may be depicted in the drawings or described in the specification in a particular order, such operations need not be performed in the particular order shown or in sequential order, or that all operations be performed, to achieve desirable results. Other operations that are not depicted or described can be incorporated in the example methods and processes. For example, one or more additional operations can be performed before, after, simultaneously, or between any of the described operations. Further, the operations may be rearranged or reordered in other implementations. Those skilled in the art will appreciate that in some embodiments, the actual steps taken in the processes illustrated and/or disclosed may differ from those shown in the figures. Depending on the embodiment, certain of the steps described above may be removed, others may be added. Furthermore, the features and attributes of the specific embodiments disclosed above may be combined in different ways to form additional embodiments, all of which fall within the scope of the present disclosure. Also, the separation of various system components in the implementations described above should not be understood as requiring such separation in all implementations, and it should be understood that the described components and systems can generally be integrated together in a single product or packaged into multiple products.

For purposes of this disclosure, certain aspects, advantages, and novel features are described herein. Not necessarily all such advantages may be achieved in accordance with any particular embodiment. Thus, for example, those skilled in the art will recognize that the disclosure may be embodied or carried out in a manner that achieves one advantage or a group of advantages as taught herein without necessarily achieving other advantages as may be taught or suggested herein.

Conditional language, such as "can," "could," "might," or "may," unless specifically stated otherwise, or otherwise understood within the context as used, is generally intended to convey that certain embodiments include, while other embodiments do not include, certain features, elements, and/or steps. Thus, such conditional language is not generally intended to imply that features, elements, and/or steps are in any way required for one or more embodiments or that one or more embodiments necessarily include logic for deciding, with or without user input or prompting, whether these features, elements, and/or steps are included or are to be performed in any particular embodiment.

Conjunctive language such as the phrase "at least one of X, Y, and Z," unless specifically stated otherwise, is otherwise understood with the context as used in general to convey that an item, term, etc. may be either X, Y, or Z. Thus, such conjunctive language is not generally intended to imply that certain embodiments require the presence of at least one of X, at least one of Y, and at least one of Z.

Language of degree used herein, such as the terms "approximately," "about," "generally," and "substantially" as used herein represent a value, amount, or characteristic close to the stated value, amount, or characteristic that still performs a desired function or achieves a desired result. For example, the terms "approximately", "about", "generally," and "substantially" may refer to an amount that is within less than 10% of, within less than 5% of, within less than 1% of, within less than 0.1% of, and within less than 0.01% of the stated amount. As another example, in certain embodiments, the terms "generally parallel" and "substantially parallel" refer to a value, amount, or characteristic that departs from exactly parallel by less than or equal to 15 degrees, 10 degrees, 5 degrees, 3 degrees, 1 degree, or 0.1 degree.

The scope of the present disclosure is not intended to be limited by the specific disclosures of preferred embodiments in this section or elsewhere in this specification and may be defined by claims as presented in this section or elsewhere in this specification or as presented in the future. The language of the claims is to be interpreted broadly based on the language employed in the claims and not limited to the examples described in the present specification or during the prosecution of the application, which examples are to be construed as non-exclusive.

Of course, the foregoing description is that of certain features, aspects and advantages of the present disclosure, to which various changes and modifications can be made without departing from the scope of the present invention. Moreover, the devices described herein need not feature all of the objects, advantages, features and aspects discussed above. Thus, for example, those of skill in the art will recognize that the invention can be embodied or carried out in a manner that achieves or optimizes one advantage or a group of advantages as taught herein without necessarily achieving other objects or advantages as may be taught or suggested herein. In addition, while a number of variations of the invention have been shown and described in detail, other modifications and methods of use, which are within the scope of this invention, will be readily apparent to those of skill in the art based upon this disclosure. It is contemplated that various combinations or subcombinations of these specific features and aspects of embodiments may be made and still fall within the scope of the invention. Accordingly, it should be understood that various features and aspects of the disclosed embodiments can be combined with or substituted for one another in order to form varying modes of the discussed devices.

## Claims

1. A system for controlling a prosthetic or orthotic device, comprising:
an actuator (101) configured to rotate in an anterior-posterior direction about a medial-lateral axis, the actuator (101) comprising a magnetorheological (MR) fluid and a coil (814) operable to selectively apply a magnetic field to the MR fluid to vary its viscosity and thereby vary a resistive torque of the actuator (101) about the medial-lateral axis;
one or more sensors (204, 205, 206) embedded in or attached to the prosthetic device; and
circuitry configured to implement a control system architecture (850) to control an amplitude of a current applied to the coil (814) to vary the resistive torque of the actuator based on data collected from the one or more sensors (204, 205, 206), **characterized by** the control system architecture including a Luenberger observer (803) operable to generate an actuator velocity estimate, wherein the actuator velocity estimate is used in a closed-loop velocity control to regulate the resistive torque generated by the MR actuator.

2. The system of Claim 1, wherein the control system architecture includes an inference layer (301) and a reactive layer (302).

3. The system of Claim 2, wherein the reactive layer (302) implements a state machine (406) comprising two subphases associated with a swing phase (501) and three subphases associated with a stance phase (502), wherein:
the swing phase (501) defines a state where the prosthetic or orthotic device is not carrying a user's weight or in contact with a ground surface, and
the stance phase (502) defines a state where the prosthetic or orthotic device is carrying a user's weight or in contact with the ground surface.

4. The system of any of Claims 1-3, wherein the actuator velocity estimate is used to distinguish between a swing flexion subphase and a swing extension subphase.

5. The system of any of Claims 1-4, wherein the control system architecture includes an Angle Dependent Component setpoint generation (804) configured to define a relationship between a position of the actuator (101) and at least a portion of a resistive torque set-point.

6. The system of Claim 5, wherein the Angle Dependent Component setpoint generation (804) is defined such that a contribution to the resistive torque set-point for the actuator (101) linearly increases over approximately a first half of an actuator motion range and then linearly decreases over a remaining actuator motion range.

7. The system of Claim 5, wherein the Angle Dependent Component setpoint generation (804) is defined such that a contribution to the resistive torque set-point for the actuator (101) linearly increases over an entire actuator motion range.

8. The system of Claim 7, wherein the control system architecture (804) includes a velocity control loop for controlling a torsional resistance of the actuator, the velocity control loop comprising a heuristic torque set-point generation module including the Angle Dependent Component setpoint generation (804) and a static component.

9. The system of Claim 5, wherein where the Angle Dependent Component setpoint generation (804) is defined in such a way as to exponentially increase its resistive torque contribution to the actuator set-point over a complete actuator motion range.

10. The system of Claim 5, wherein the Angle Dependent Component setpoint generation (804) is defined such that a contribution to the resistive torque set-point for the actuator (101) asymptotically increases towards a predefined resistive torque contribution over an entire actuator motion range.

11. The system of Claim 5, wherein the Angle Dependent Component setpoint generation (804) is defined such that a contribution to the resistive torque set-point for the actuator (101) presents a M-profiled resistive torque contribution over an entire actuator motion range.

## Patentansprüche

1. System zur Steuerung einer Prothese- oder Orthesevorrichtung, umfassend:
eine Betätigungsvorrichtung (101), die so konfiguriert ist, dass sie sich in einer anteriorposterioren Richtung um eine medial-laterale Achse dreht, wobei die Betätigungsvorrichtung (101) ein magnetorheologisches (MR) Fluid und eine Spule (814) umfasst, die dazu dient, selektiv ein Magnetfeld an das MR-Fluid anzulegen, um dessen Viskosität zu verändern und dadurch ein Widerstandsdrehmoment der Betätigungsvorrichtung (101) um die medial-laterale Achse zu verändern;
einen oder mehrere Sensoren (204, 205, 206), die in die Prothesevorrichtung eingebettet oder an ihr angebracht sind; und
Schaltung, die so konfiguriert ist, dass sie eine Steuerungssystemarchitektur (850) implementiert, um eine Amplitude eines an die Spule (814) angelegten Stroms zu steuern, um das Widerstandsdrehmoment der Betätigungsvorrichtung auf der Grundlage von Daten zu variieren, die von dem einen oder den mehreren Sensoren (204, 205, 206) erfasst werden, **dadurch gekennzeichnet, dass** die Steuerungssystemarchitektur einen Luenberger-Beobachter (803) beinhaltet, der in der Lage ist, eine Schätzung der Geschwindigkeit der Betätigungsvorrichtung zu generieren, wobei die Schätzung der Geschwindigkeit der Betätigungsvorrichtung in einer geschlossenen Geschwindigkeitssteuerung verwendet wird, um das von der MR-Betätigungsvorrichtung generierte Widerstandsdrehmoment zu steuern.

2. System nach Anspruch 1, wobei die Steuerungssystemarchitektur eine Inferenzschicht (301) und eine reaktive Schicht (302) beinhaltet.

3. System nach Anspruch 2, wobei die reaktive Schicht (302) eine Zustandsmaschine (406) implementiert, die zwei Teilphasen umfasst, die mit einer Schwungphase (501) verbunden sind, und drei Teilphasen, die mit einer Standphase (502) verbunden sind, wobei:
die Schwungphase (501) einen Zustand definiert, in dem die Prothese- oder Orthesevorrichtung weder ein Gewicht des Benutzers trägt noch in Kontakt mit einem Boden steht, und
die Standphase (502) einen Zustand definiert, in dem die Prothese- oder Orthesevorrichtung ein Gewicht des Benutzers trägt oder in Kontakt mit einem Boden steht.

4. System nach einem der Ansprüche 1-3, wobei die Schätzung der Geschwindigkeit der Betätigungsvorrichtung dazu dient, zwischen einer Schwungbeuge-Teilphase und einer Schwungstreckungs-Teilphase zu unterscheiden.

5. System nach einem der Ansprüche 1-4, wobei die Architektur des Steuerungssystems eine Sollwertgenerierung der winkelabhängigen Komponente (804) beinhaltet, die so konfiguriert ist, dass sie eine Beziehung zwischen einer Position der Betätigungsvorrichtung (101) und mindestens einem Teil eines Sollwerts des Widerstandsdrehmoments definiert.

6. System nach Anspruch 5, wobei die Sollwertgenerierung der winkelabhängigen Komponente (804) so definiert ist, dass ein Beitrag zum Sollwert des Widerstandsdrehmoments für die Betätigungsvorrichtung (101) über etwa die erste Hälfte eines Bewegungsbereichs der Betätigungsvorrichtung linear ansteigt und dann über den verbleibenden Bewegungsbereich der Betätigungsvorrichtung linear abnimmt.

7. System nach Anspruch 5, wobei die Sollwertgenerierung der winkelabhängigen Komponente (804) so definiert ist, dass ein Beitrag zum Sollwert des Widerstandsdrehmoments für die Betätigungsvorrichtung (101) über den gesamten Bewegungsbereich der Betätigungsvorrichtungs linear ansteigt.

8. System nach Anspruch 7, wobei die Steuerungssystemarchitektur (804) einen Geschwindigkeitssteuerkreis zur Steuerung eines Torsionswiderstands der Betätigungsvorrichtung beinhaltet, wobei der Geschwindigkeitsregelkreis ein heuristisches Drehmoment-Sollwertgenerierungsmodul umfasst, das die Sollwertgenerierung der winkelabhängigen Komponente (804) und eine statischen Komponente beinhaltet.

9. System nach Anspruch 5, wobei die Sollwertgenerierung der winkelabhängigen Komponente (804) so definiert ist, dass ihr Widerstandsdrehmomentbeitrag zum Sollwert der Betätigungsvorrichtung über einen gesamten Bewegungsbereich der Betätigungsvorrichtung exponentiell ansteigt.

10. System nach Anspruch 5, wobei die Sollwertgenerierung der winkelabhängigen Komponente (804) so definiert ist, dass ein Beitrag zum Sollwert des Widerstandsdrehmoments für die Betätigungsvorrichtung (101) über den gesamten Bewegungsbereich der Betätigungsvorrichtung asymptotisch auf einen vordefinierten Widerstandsdrehmomentbeitrag ansteigt.

11. System nach Anspruch 5, wobei die Sollwertgenerierung der winkelabhängigen Komponente (804) so definiert ist, dass ein Beitrag zum Sollwert des Widerstandsdrehmoments für die Betätigungsvorrichtung (101) einen M-förmigen Widerstandsdrehmomentbeitrag über einen gesamten Bewegungsbereich der Betätigungsvorrichtung darstellt.

## Revendications

1. Système de commande d'un dispositif prothétique ou orthétique, comprenant :
un actionneur (101) configuré pour tourner dans une direction antéro-postérieure autour d'un axe médio-latéral, l'actionneur (101) comprenant un fluide magnétorhéologique (MR) et une bobine (814) conçue pour appliquer sélectivement un champ magnétique au fluide MR afin de faire varier sa viscosité et ainsi faire varier un couple de résistance de l'actionneur (101) autour de l'axe médio-latéral ;
un ou plusieurs capteurs (204, 205, 206) intégrés ou fixés au dispositif prothétique ; et
un ensemble de circuits configuré pour mettre en œuvre une architecture de système de commande (850) pour commander l'amplitude d'un courant appliqué à la bobine (814) afin de faire varier le couple de résistance de l'actionneur en fonction des données collectées à partir des un ou plusieurs capteurs (204, 205, 206), **caractérisé par** l'architecture de système de commande incluant un observateur de Luenberger (803) conçu pour générer une estimation de la vitesse de l'actionneur, dans laquelle l'estimation de la vitesse de l'actionneur est utilisée dans une commande de vitesse en boucle fermée pour réguler le couple de résistance généré par l'actionneur MR.

2. Système selon la revendication 1, dans lequel l'architecture de système de commande inclut une couche d'inférence (301) et une couche réactive (302).

3. Système selon la revendication 2, dans lequel la couche réactive (302) met en œuvre une machine à états (406) comprenant deux sous-phases associées à une phase d'oscillation (501) et trois sous-phases associées à une phase d'appui (502), dans lequel :
la phase d'oscillation (501) définit un état dans lequel le dispositif prothétique ou orthétique ne supporte pas le poids de l'utilisateur ou n'est pas en contact avec une surface du sol, et
la phase d'appui (502) définit un état dans lequel le dispositif prothétique ou orthétique supporte le poids de l'utilisateur ou est en contact avec la surface du sol.

4. Système selon l'une quelconque des revendications 1-3, dans lequel l'estimation de la vitesse de l'actionneur est utilisée pour faire la distinction entre une sous-phase de flexion d'oscillation et une sous-phase d'extension d'oscillation.

5. Système selon l'une quelconque des revendications 1-4, dans lequel l'architecture de système de commande inclut une génération de point de consigne de composant dépendant de l'angle (804) configurée pour définir une relation entre une position de l'actionneur (101) et au moins une partie d'un point de consigne de couple de résistance.

6. Système selon la revendication 5, dans lequel la génération de point de consigne de composant dépendant de l'angle (804) est définie de telle sorte qu'une contribution au point de consigne de couple de résistance pour l'actionneur (101) augmente linéairement sur environ la première moitié d'une plage de mouvement de l'actionneur, puis diminue linéairement sur la plage de mouvement restante de l'actionneur.

7. Système selon la revendication 5, dans lequel la génération du point de consigne de composant dépendant de l'angle (804) est définie de telle sorte qu'une contribution au point de consigne de couple de résistance pour l'actionneur (101) augmente linéairement sur toute la plage de mouvement de l'actionneur.

8. Système selon la revendication 7, dans lequel l'architecture de système de commande (804) inclut une boucle de commande de vitesse pour commander une résistance à la torsion de l'actionneur, la boucle de commande de vitesse comprenant un module de génération de point de consigne de couple heuristique incluant la génération de point de consigne de composant dépendant de l'angle (804) et un composant statique.

9. Système selon la revendication 5, dans lequel la génération de point de consigne de composant dépendant de l'angle (804) est définie de manière à augmenter exponentiellement sa contribution de couple de résistance au point de consigne de l'actionneur sur une plage de mouvement complète de l'actionneur.

10. Système selon la revendication 5, dans lequel la génération de point de consigne de composant dépendant de l'angle (804) est définie de telle sorte qu'une contribution au point de consigne de couple de résistance pour l'actionneur (101) augmente asymptotiquement vers une contribution de couple de résistance prédéfinie sur toute la plage de mouvement de l'actionneur.

11. Système selon la revendication 5, dans lequel la génération de point de consigne de composant dépendant de l'angle (804) est définie de telle sorte qu'une contribution de point de consigne de couple de résistance pour l'actionneur (101) présente une contribution de couple de résistance à profil en M sur toute la plage de mouvement de l'actionneur.
